# EUROPÄISCHE PATENTSCHRIFT

(11) **EP 2 919 846 B1**
(45) Veröffentlichungstag und Bekanntmachung des Hinweises auf die Patenterteilung: **10.04.2019**
(21) Anmeldenummer: 14707358.9
(22) Anmeldetag: 25.02.2014
(51) Int. Cl.: A61M 25/00

(54) **KATHETER, INSBESONDERE ZUR BEHANDLUNG VON PROSTATA UND/ODER BLASE, UND DIESEN KATHETER ENTHALTENDES KIT**
CATHETER, IN PARTICULAR FOR TREATING THE PROSTATE AND/OR BLADDER, AND KIT CONTAINING SAID CATHETER
CATHÉTER, EN PARTICULIER DESTINÉ AU TRAITEMENT DE LA PROSTATE ET/OU DE LA VESSIE, ET KIT CONTENANT CE CATHÉTER

(30) Priorität: 27.02.2013 DE 102013003221; 04.03.2013 DE 102013003517
(43) Veröffentlichungstag der Anmeldung: 23.09.2015
(73) Patentinhaber: Otto, Ullrich, 34537 Bad Wildungen (DE)
(72) Erfinder: Otto, Ullrich, 34537 Bad Wildungen (DE)
(74) Vertreter: Strehlke, Ingo Kurt
(86) Internationale Anmeldenummer: PCT/EP2014/053586
(87) Internationale Veröffentlichungsnummer: WO 2014/131742

(56) Entgegenhaltungen:
- WO-A2-03/033045
- DE-A1-102011 110 778
- DE-U1-202011 104 675
- US-A- 5 007 897
- US-A1- 2002 165 521
- US-A1- 2010 010 470

## Beschreibung

Die vorliegende Erfindung betrifft das technische Gebiet der Behandlung von Funktionsstörungen und/oder Erkrankungen der Prostata und gegebenenfalls der Blase, insbesondere zur medikamentösen und/oder physikalischen Behandlung der Prostata und gegebenenfalls der Blase, vorzugsweise in Verbindung mit oder nach einer transurethralen Resektion und/oder Vaporisation der Prostata, in Verbindung mit einer benignen Prostatahyperplasie oder in Verbindung mit oder nach einer Bestrahlungstherapie eines Karzinoms der Prostata.

Insbesondere betrifft die vorliegende Erfindung einen Katheter, in Form eines Verweilkatheters, welcher sich bevorzugt zur Behandlung von Funktionsstörungen bzw. Erkrankungen der Prostata und gegebenenfalls der Blase, insbesondere zur medikamentösen und/oder physikalischen Behandlung der Prostata und gegebenenfalls der Blase, vorzugsweise in Verbindung mit oder nach einer transurethralen Resektion bzw. Vaporisation der Prostata, in Verbindung mit einer benignen Prostatahyperplasie oder in Verbindung mit oder nach einer Bestrahlungstherapie eines Prostatakarzinoms, eignet, sowie ein diesen Katheter enthaltendes Kit (Set) in Form eines Kathetersystems.

Das Prostatakarzinom stellt den häufigsten Tumor des Mannes dar. Aktuell werden in Deutschland pro Jahr ca. 64.000 Neuerkrankungen diagnostiziert. Durch die bessere Diagnostik wird das Prostatakarzinom immer häufiger im lokalisierten Stadium diagnostiziert, so dass die Möglichkeit einer Kuration gegeben ist. In der Vergangenheit bestand die Therapie des lokalisierten Prostatakarzinoms nahezu ausschließlich in der radikalen Prostatovesikulektomie.

In den letzten Jahren sind verschiedene konkurrierende Therapieverfahren entwickelt worden, die zu einer veränderten Patientenpräferenz geführt haben. Dabei setzten sich vor allem diejenigen Therapiemaßnahmen durch, bei denen es mit Hilfe von technologisch anspruchsvollen Maßnahmen gelingt, Karzinomgewebe präzise und punktgenau zu destruieren.

Aufgrund evidenzbasierter Daten konnten sich besonders radiologische Behandlungsverfahren etablieren. Sie erzielen nachweislich eine befriedigende Kontrolle des Tumors.

Die interdisziplinären Leitlinien zur Früherkennung (insbesondere der Qualität S3), Diagnose und Therapie der verschiedenen Stadien des Prostatakarzinoms empfehlen daher auch radiologische Therapieverfahren als primäre Behandlung des lokalisierten Prostatakarzinoms. Aus den vorhandenen Literaturdaten lässt sich erkennen, dass der Anteil der radiotherapierten Patienten derzeit ca. 30 % beträgt. In Amerika ist dieses Therapieverfahren seit Jahren etabliert. Dort werden pro Jahr ca. 80.000 von ca. 240.000 neu erkrankten Patienten mit einem lokalisierten Prostatakarzinom bestrahlt.

Allerdings führt die Bestrahlungstherapie zu postradiogenen Funktionsstörungen der Blase und/oder der Prostata, welche die Strahlendosis limitiert und die Lebensqualität des Patienten einschränkt.

Die Hauptfunktionsstörungen betreffen die Blasenfunktion. Diese Störungen sind gekennzeichnet durch eine irritative und obstruktive Symptomatik der Prostata und der Blase. Insbesondere werden die Prostata- und die Blasenschleimhaut in Mitleidenschaft gezogen. Die Bestrahlung führt zu einer Reizung der Prostata- und Blasenschleimhaut und vermehrtem Harndrang mit einer irritativen Symptomatik. Im Ergebnis kommt es zu einer Störung der Blasenspeicherphase, welche eine erhebliche Drangsymptomatik verursacht. Zusätzlich kann es durch ein Ödem der bestrahlten Prostata zu einer Obstruktion kommen. Irritative und obstruktive Komponenten führen zu einer Symptomatik, welche eine Pollakisurie, Nykturie, Dysurie und Restharnbildung bis zum Harnverhalt beinhaltet.

Die vorgenannten Funktionsstörungen der Blase und/oder Prostata werden zur Zeit zum einen medikamentös, vorwiegend per os, also systemisch, oder durch intravesikale Therapien behandelt, wobei jedoch infolgedessen die Konzentration der verabreichten Substanzen am Erfolgsorgan gering und/oder deren Verweildauer kurz ist. Folglich sind oftmals sehr hohe Dosen der verabreichten Substanzen erforderlich, was jedoch zu unerwünschten systemischen Nebenwirkungen führen kann.

Weiterhin steht bei Männern ab dem 50. Lebensjahr nicht nur das Prostatakarzinom, sondern auch die Vergrößerung der Prostata als Erkrankung im Vordergrund. In diesem Alter treten die ersten Anzeichen der gutartigen Vergrößerung auf, welche als sogenannte benigne Prostatahyperplasie (BPH) bezeichnet wird. Diese benigne Prostatahyperplasie kann bei älteren Männern als "Volkskrankheit" bezeichnet werden. Die Prävalenz der benignen Prostatahyperplasie (BPH) nimmt mit dem Alter deutlich zu. Während in Autopsie-Studien gezeigt werden konnte, dass bei Männern unter 30 Jahren keine benigne Prostatahyperplasie (BPH) diagnostiziert wurde, liegt bei Männern im Alter von 80 Jahren in 88 % der Fällen eine benigne Prostatahyperplasie (BPH) vor (T. Bach, M. S. Michel, Benignes Prostatasyndrom (BPS), Hautmann, Urologie, 4. Auflage, Springer-Verlag, S. 178).

Durch die Vergrößerung der Prostata komprimiert diese die Harnröhre, und es kommt zu klinisch relevanten Folgen bzw. Funktionsstörungen. Die so entstehenden klinischen Symptome kann man in irritative und obstruktive Symptome aufgliedern. Unter den irritativen Symptomen subsumiert sich die Pollakisurie (häufiges Miktionieren am Tage), die Drangsymptomatik und die Nykturie (nächtliches Miktionieren). Die obstruktiven Symptome manifestieren sich durch einen abgeschwächten Harnstrahl, eine prolongierte Miktion, "Startschwierigkeiten", Harnträufeln und "Restharngefühle".

Werden diese Symptome nicht behandelt und somit beseitigt, kann es zu einem Harnverhalt, zu einer "Überlaufblase", zu einer Blasensteinbildung, zu einer Detrusordekompensation (Versagen des Austragungsmuskels), zu einer Hydronephrose (Stauung der Nieren) oder zu einer postrenalen Niereninsuffizienz, teilweise mit schwerwiegenden oder sogar lebensbedrohlichen Folgen, kommen.

Für die Therapie der benignen Prostatahyperplasie (BPH) stehen verschiedene Behandlungsmöglichkeiten zur Verfügung, nämlich die einfache Überwachung bei Patienten mit "milden" Symptomen, medikamentöse Therapien sowie operativen Therapien (T. Bach, M. S. Michel, Benignes Prostatasyndrom (BPS), Hautmann, Urologie, 4. Auflage, Springer-Verlag, S. 183).

Die Indikation zur Einleitung einer Behandlung hängt von der Schwere der Symptome sowie evtl. auftretenden Komplikationen ab.

Die transurethrale Resektion der Prostata (TUR-P) gilt nach wie vor als Standard in der instrumentellen Therapie der gutartigen Prostatavergrößerung und wird bei 95 % aller chirurgischen Eingriffe zur Behandlung der benignen Prostatahyperplasie (BPH) angewandt.

Die transurethrale Resektion der Prostata (TUR-P) wird in der Bundesrepublik bei ca. 60.000 Patienten pro Jahr und in Europa bei ca. 300.000 Patienten pro Jahr durchgeführt. Die Komplikationen bestehen in signifikanten Hyponatriämien, einem TUR-Syndrom, therapiebedürftigen Blutungen, Bluttransfusionen, Harnwegsinfekten, Epididymitis, Inkontinenz, Blasenhalssklerosen, Harnröhrenstrikturen, retrograden Ejakulationen und Impotenz (A. Leyh, R. Hartung, Transurethrale Elektroresektion der Prostata (TUR-P), in: K. Höfner, C. G. Stief, U. Jonas, Benigne Prostatahyperplasie: Ein Leitfaden für die Praxis, Springer-Verlag, Seite 476).

Sowohl bei der transurethralen Resektion der Prostata (TUR-P) als auch bei der Vaporisierung entsteht im Operationsgebiet eine große Wundfläche. Diese Wundfläche führt in der differenzierten Betrachtung dazu, dass es vor allen Dingen in der Langzeitbeobachtung (mindestens 30 Tage) zu einer ausgeprägten irritativen Symptomatik, zu einer vorübergehenden Inkontinenz sowie zu Blasenspasmen und Dysurien kommt. Beurteilt man den Therapieerfolg einer transurethralen Resektion oder Vaporisation mit Hilfe eines Symptomen-Score, so muss darüber hinaus festgestellt werden, dass die Symptomminderung erst nach sechs Monaten ihr Optimum erreicht hat.

Die Lebensqualität der transurethral rezesierten Patienten ist also insbesondere aufgrund der irritativen Symptomatik deutlich eingeschränkt. Die vorgenannte irritative Symptomatik wird zur Zeit zum einen medikamentös, vorwiegend per os, also systemisch, oder durch intravesikale Therapien behandelt, wobei jedoch infolgedessen die Konzentration der verabreichten Substanzen am Erfolgsorgan gering und/oder deren Verweildauer kurz ist. Folglich sind oftmals sehr hohe Dosen der verabreichten Substanzen erforderlich, was jedoch zu unerwünschten systemischen Nebenwirkungen führen kann.

Aus der WO 03/033045 A2 ist ein Katheter zur Behandlung von Funktionsstörungen der Prostata sowie zur Entnahme von Prostataflüssigkeit bekannt. Jedoch ist in diesem Dokument lediglich eine physikalische Behandlung der Prostata mittels Kompression bzw. Druck und Wärme vorgesehen, da keine Medikamentenabgabe aus dem Katheter erfolgen kann.

Weiterhin beschreibt die US 2002/0165521 A1 einen Katheter zur Behandlung der Prostata mit einem Prostataballon, wobei der Prostataballon impermeabel ausgebildet ist, aber zusätzlich mit einer porösen elastischen Membran überzogen ist, welche die Abgabe von Medikamenten aus dem Zwischenraum zwischen Prostataballon und poröser Membran ermöglichen soll. Das Medikament wird bereits bei der Herstellung des Katheters in den Zwischenraum zwischen Prostataballon und poröser Membran eingebracht, so dass aufwendige Sterilisationsmaßnahmen erforderlich sind und der Katheter nur eine begrenzte Haltbarkeit aufweist, da die Befüllung bereits vor Instillation des Katheters erfolgen muss. Außerdem können durch die poröse Membran bereits vor Benutzung des Katheters größere Mengen an Medikament austreten. Zudem ist auch eine nachträgliche Zugabe des Medikaments im eingeführten Zustand des Katheters nicht mehr möglich.

Schließlich betreffen die auf den Anmelder selbst zurückgehende DE 10 2011 110, die als der nächstliegende Stand der Technik angesehen wird, sowie das zugehörige Patentfamilienäquivalent WO 2013/010600 A1 einen Katheter, insbesondere Verweilkatheter, welcher sich bevorzugt zur Behandlung von Funktionsstörungen oder Erkrankungen der Blase und gegebenenfalls der Prostata eignet. Es wird maßgeblich auf die Behandlung der Blase nach einer Bestrahlungstherapie eines Prostatakarzinoms abgestellt. Grundsätzlich ist jedoch auch die medikamentöse Behandlung der Prostata vorgesehen, wobei zu diesem Zweck drei gleichrangige Ansätze offenbart werden, nämlich eine außenseitige Beschichtung des Prostataballons mit einem Medikament, eine zumindest außenseitige Inkorporierung eines Medikaments zur Behandlung der Prostata in die Wandung des Prostataballons oder eine permeable oder semipermeable Ausbildung der Wandung des prostatischen Ballons zur Medikamentenabgabe, wobei sich diesbezüglich keinerlei konkrete Angaben finden lassen.

Der vorliegenden Erfindung liegt daher die Aufgabe zugrunde, einen Katheter zur Behandlung von Funktionsstörungen bzw. Erkrankungen der Prostata und gegebenenfalls der Blase sowie ein diesen Katheter enthaltendes Kit (Kit-of-parts) bzw. Set bereitzustellen, wobei die zuvor geschilderten, mit dem Stand der Technik verbundenen Nachteile zumindest weitgehend vermieden oder aber wenigstens abgeschwächt werden sollen.

Insbesondere ist eine Aufgabe der vorliegenden Erfindung die Bereitstellung eines Katheters, in Form eines Verweilkatheters, sowie ein diesen Katheter enthaltendes applikationsbereites Kit (Kit-of-parts) bzw. Set, in Form eines Kathetersystems, wobei eine Eignung zur medikamentösen und/oder physikalischen Behandlung der Prostata und gegebenenfalls der Blase gegeben sein soll. Insbesondere sollen unerwünschte medikamentös bedingte Nebenwirkungen, wie sie bei einer systemischen Verabreichung von Medikamenten auftreten, zumindest weitestgehend vermieden werden.

Weiterhin ist eine Aufgabe der vorliegenden Erfindung die Bereitstellung eines Katheters, in Form eines Verweilkatheters, sowie ein diesen Katheter enthaltendes applikationsbereites Kit (Kit-of-parts) bzw. Set in Form eines Kathetersystems, wobei eine Eignung zur Behandlung von Funktionsstörungen bzw. Erkrankungen der Prostata und gegebenenfalls der Blase in Verbindung mit oder nach einer transurethralen Resektion bzw. Vaporisation der Prostata, in Verbindung mit einer benignen Prostatahyperplasie oder in Verbindung mit oder nach einer Bestrahlungstherapie eines Prostatakarzinoms gegeben sein soll.

Zur Lösung des zuvor geschilderten Problems schlägt die vorliegende Erfindung somit einen Katheter, in Form eines Verweilkatheters, nach Anspruch 5 vor. Weitere, vorteilhafte Eigenschaften des erfindungsgemäßen Katheters sind Gegenstand der diesbezüglichen Unteransprüche.

Weiterer Gegenstand der vorliegenden Erfindung ist ein Kit (d. h. ein Kit-of-parts oder Set), in Form eines Kathetersystems, nach Anspruch 1, welches einerseits den erfindungsgemäßen Katheter sowie andererseits mindestens eine mindestens ein Medikament enthaltende Applikationsvorrichtung zur Verabreichung des Medikaments umfasst, wie es in den entsprechenden, auf das Kit selbst gerichteten Ansprüchen beschrieben bzw. definiert ist.

Es versteht sich von selbst, dass besondere Ausgestaltungen und Ausführungsformen, welche nur im Zusammenhang mit einem Erfindungsaspekt beschrieben sind, auch in Bezug auf die anderen Erfindungsaspekte entsprechend gelten, ohne dass dies ausdrücklich beschrieben ist.

Im Übrigen gilt, dass der Fachmann anwendungsbezogen oder einzelfallbedingt von den nachfolgend aufgeführten Zahlen-, Wert- bzw. Bereichsangaben abweichen kann, ohne dass er den Rahmen der vorliegenden Erfindung verlässt.

Gegenstand der vorliegenden Erfindung - gemäß einem **ersten** Aspekt der vorliegenden Erfindung - ist somit ein Katheter mit den Merkmalen des Anspruchs 5.

Bei dem erfindungsgemäßen Katheter handelt es sich um einen speziellen Verweilkatheter, mit welchem sowohl durch physikalische als auch durch medikamentöse Maßnahmen nicht nur Funktionsstörungen der Prostata und gegebenenfalls der Blase, sondern auch Erkrankungen, wie Prostatakarzinome, gutartige Vergrößerungen der Prostata, Blasenkarzinome oder dergleichen, behandelt werden können. Insbesondere eignet sich der erfindungsgemäße Katheter zur Behandlung der Prostata und gegebenenfalls der Blase in Verbindung mit oder nach einer transurethralen Resektion bzw. Vaporisation der Prostata oder in Verbindung mit oder nach einer Bestrahlungstherapie eines Karzinoms der Prostata.

Dabei sollte der erfindungsgemäße Katheter, insbesondere Verweilkatheter, eine Verweildauer von maximal 60 Stunden, insbesondere maximal 48 Stunden, haben, obwohl kürzere oder längere Verweildauern nicht ausgeschlossen sind.

Der erfindungsgemäß vorhandene Prostataballon dient zum einen der Kompression und damit der lokalen physikalischen Behandlung der Prostata. Durch die mittels Poren permeabel ausgebildete Wandung lässt sich zudem ein in den Prostataballon eingebrachtes Medikament gezielt und lokal beschränkt in den zu behandelnden Prostatabereich abgeben, so dass gleichermaßen eine effiziente medikamentöse Behandlung stattfinden kann, wobei durch die topische bzw. lokale Abgabe des Medikaments eine hohe lokale Wirkstoffkonzentration im Vergleich zu einer systemischen Gabe erreicht werden kann, obwohl aufgrund der lokalen Applikation die absolute Wirkstoffmenge signifikant reduziert werden kann. Üblicherweise erfolgt die Abgabe des Medikaments durch die permeabel ausgebildete Wandung des Prostataballons zumindest im Wesentlichen über Diffusionsprozesse, d. h. das Medikament diffundiert durch die permeabel ausgebildete Wandung des Prostataballons gezielt in den zu behandelnden Prostatabereich, wobei über die Durchlässigkeit der Wandung des Prostataballons die Abgabemenge des Medikaments pro Zeiteinheit exakt gesteuert werden kann. Letztendlich wird durch den befüllten Prostataballon die Harnröhre im Bereich der Prostata aufgeweitet und medikamentös beeinflusst bzw. behandelt, d. h. der erfindungsgemäße Katheter ermöglicht einerseits eine physikalische Behandlung, insbesondere mittels Kompression, und andererseits eine effiziente pharmakologische Behandlung durch gezielte Medikamentenabgabe.

Um eine effiziente Medikamentenabgabe zu ermöglichen, weist die permeabel ausgebildete Wandung des Prostataballons im Allgemeinen eine Mehrzahl von Poren auf. Die Anzahl an Poren kann in weiten Bereichen variieren. Im Allgemeinen weist die Wandung des Prostataballons eine Porenanzahl von 1 bis 1.000 Poren, insbesondere 1 bis 500 Poren, vorzugsweise 2 bis 100 Poren, bevorzugt 2 bis 50 Poren, besonders bevorzugt 2 bis 20 Poren, ganz besonders bevorzugt 3 bis 10 Poren, insbesondere bevorzugt 4 bis 6 Poren, auf. Auf diese Weise wird eine optimale Wirkstoffkonzentration am Behandlungsort im Prostatabereich erzielt.

Besonders gute Ergebnisse werden erfindungsgemäß erhalten, wenn die permeabel ausgebildete Wandung des Prostataballons im expandierten Zustand Poren mit einer Porengröße, insbesondere mit einem Durchmesser, im Bereich von 1 bis 100 µm, insbesondere 2 bis 80 µm, vorzugsweise 3 bis 60 µm, bevorzugt 4 bis 50 µm, besonders bevorzugt 5 bis 40 µm, ganz besonders bevorzugt 8 bis 30 µm, noch mehr bevorzugt 15 bis 25 µm, aufweist. Auf diese Weise wird insbesondere gewährleistet, dass das abzugebende Medikament zumindest im Wesentlichen über diffusionsgesteuerte Prozesse freigesetzt wird und eine übermäßig schnelle Abgabe des freizusetzenden Medikaments verhindert wird, d. h. auf diese Weise wird der Medikamententransport über physikalische Prozesse gesteuert.

Erfindungsgemäß besonders gute Ergebnisse werden erhalten, wenn die Poren der permeabel ausgebildeten Wandung des Prostataballons durch Laserperforation, insbesondere mittels eines Ultrakurzpulslasers, erzeugt sind. Auf diese Weise kann eine definierte und vorgegebene Porengröße sozusagen maßgeschneidert eingestellt werden, ohne dass das verbleibende Material des Prostataballons beeinträchtigt wird. Durch die Anwendung eines Lasers zur Erzeugung der Poren werden besonders scharf begrenzte Ränder erhalten. Insbesondere wird das Kunststoffmaterial des Prostataballons derart perforiert, dass die Ränder der Poren bei ihrer Erzeugung gleichsam verschweißt werden und nicht einreißen können. Wie der Anmelder überraschenderweise herausgefunden hat, führen alternative Methoden zur Erzeugung der Poren, insbesondere durch mechanische Bohrung, Inkorporation von Fremdkörpern und nachfolgende Verstreckung oder chemisches Entfernen der Fremdkörper oder anderer Bestandteile mittels Ätzen, Lösen oder dergleichen, nicht zu den gewünschten Ergebnissen.

Ganz besonders gute Ergebnisse werden erhalten, wenn die Poren der Wandung des Prostataballons durch Laserperforation mittels eines sogenannten Ultrakurzpulslasers erzeugt werden. Auf diese Weise wird das zu perforierende Kunststoffmaterial des Prostataballons nur äußerst kurzzeitig einer Laserbehandlung ausgesetzt, was besonders materialschonend ist. Im Allgemeinen werden als Ultrakurzpulslaser solche Laserstrahlquellen bezeichnet, die gepulstes Laserlicht mit Pulsdauern im Bereich von Pikosekunden, Femtosekunden und Attosekunden aussenden; es handelt sich im Allgemeinen um modengekoppelte Laser.

Bei einer nicht erfindungsgemäßen

Ausführungsform sind die Poren der Wandung des Prostataballons also *in situ* während der Herstellung der Wandung, insbesondere während der Herstellung der Wandung mittels Polymerisation und/oder mittels chemischer Modifizierung, erzeugt. Dies ist dem Fachmann als solches bekannt, so dass es diesbezüglich keiner weitergehenden Ausführungen bedarf.

Insbesondere kann im Rahmen der Herstellung der Wandung des Prostataballons mittels Polymerisation gezielt eine Erzeugung von (Mikro-)Poren realisiert werden.

Insbesondere kann dies dadurch erfolgen, dass bei der Herstellung der Wandung des Prostataballons mittels Polymerisation dem zu polymerisierenden Ausgangsgemisch der entsprechenden Monomere zusätzlich sogenannte Porenbildner oder Füllstoffe (z. B. Polyethylenglykol) zugesetzt werden, welche dann bei der Polymerisation zu gezielten Fehlstellen in Form von Mikroporen in der Membran bzw. Wandung führen. Alternativ kann im Rahmen der Herstellung der Wandung bzw. Membran des Prostataballons mittels Polymerisation aber auch eine chemische Modifizierung erfolgen (z. B. durch Zusetzen modifizierter Monomere zu der zu polymerisierenden Ausgangsmischung). Die vorgenannten Herstellungsverfahren führen zu einer gezielten Störung der erzeugten Polymermembran bzw. Wandung dahingehend, dass zielgerichtet Poren in die resultierenden Polymermembranen eingebaut werden, wobei diese Polymermembranen letztendlich als Wandung bzw. als Wandungsmaterial für den Prostataballon eingesetzt werden können.

Wie zuvor bereits ausgeführt, ist die herstellungsbedingte *in-situ*-Erzeugung von (Mikro-)Poren in Polymermembranen, insbesondere mittels Durchführung der Polymerisation in Gegenwart von sogenannten Porenbildnern oder Füllstoffen oder aber durch chemische Modifizierung, dem Fachmann als solche geläufig; es lassen sich auf diese (mikro-)poröse Membranen bzw. Membranmaterialien erzeugen, welche als Wandung für den Prostataballon eingesetzt werden können. Bezüglich der Herstellung derartiger Membranen kann rein exemplarisch und rein beispielhaft (d. h. nicht beschränkend) auf die nachfolgend genannten Dissertationen verwiesen werden, deren gesamter diesbezüglicher Offenbarungsgehalt jeweils hiermit durch Bezugnahme eingeschlossen ist: Barbara Weh, Dissertation/Würzburg 2002 (Bayerische Julius-Maximilians-Universität Würzburg) "Permeationseigenschaften von Polydimethylsiloxan-Membranen in Abhängigkeit von der Netzbogenlänge"; Claudius Pop, Dissertation/Würzburg 2006 (Bayerische Julius-Maximilians-Universität Würzburg) "Herstellung von monodispersen Polydimethylsiloxan-Netzwerken und Charakterisierung der Mikrostruktur und der Permeationseigenschaften"; Marcus Hartmann, Dissertation/Halle-Wittenberg 2005 (Martin-Luther-Universität Halle-Wittenberg) "Nichtinvasive Diffusionsuntersuchungen wässriger harnstoffhaltiger Systeme an künstlichen und biologischen Membranen"; Annina Bergner, Dissertation/Würzburg 2005 (Bayerische Julius-Maximilians-Universität Würzburg) "Charakterisierung der Mikrostruktur und der Permeationseigenschaften von Polysiloxan-Netzwerken".

Wie der Anmelder gleichermaßen überraschenderweise herausgefunden hat, liefert die herstellungsbedingte *in-situ*-Erzeugung der (Mikro-)Poren in der Wandung des Prostataballons im Rahmen der Herstellung der hierfür verwendeten Membranen vergleichbar gute Ergebnisse zur Laserperforation, da auch die herstellungsbedingt *in-situ*-generierten Poren mechanisch stabil, insbesondere einreißfest, ausgebildet sind, insbesondere im Vergleich zu nachträglich mit physikomechanischen Methoden erzeugten (Mikro-)Poren.

Durch die exakte Auswahl der Anzahl und Größe der Poren in der Wandung des Prostataballons lassen sich, insbesondere in Abhängigkeit vom zu verabreichenden Medikament, die physikalischen Transportprozesse, insbesondere die Diffusion, des zu verabreichenden Medikaments gezielt einstellen.

Im Ergebnis erfüllt der Prostataballon des erfindungsgemäßen Katheters mehrere Funktionen: Zum einen bewirkt, wie zuvor beschrieben, die Befüllung des Prostataballons eine Kompression der Prostata und eine Aufweitung der Harnröhre im Bereich der Prostata. Zum anderen ergeben sich neben dieser physikalischen Behandlung durch die Medikamentenabgabe über die durchlässige Wandung des Prostataballons die zuvor beschriebenen Vorteile. Der mit einem zu verabreichenden Medikament gefüllte Prostataballon stellt damit ein Depot bzw. ein Reservoir zur Verfügung, welches über eine längere, definierte Zeitdauer kontrolliert bzw. gesteuert eine definierte Menge eines Medikaments unmittelbar an der Therapiestelle gezielt abgibt, vorzugsweise über physikalische Prozesse, insbesondere über Diffusionsprozesse.

Im Ergebnis wird durch die vorliegende Erfindung ein transurethraler Katheter, insbesondere Verweilkatheter, zur Verfügung gestellt, welcher sich zur physikalischen wie medikamentösen lokalen bzw. topischen Therapie der Prostata und gegebenenfalls Blase, insbesondere zur Behandlung von Funktionsstörung und/oder Erkrankung von Prostata und gegebenenfalls Blase, eignet, wobei insbesondere eine medikamentöse und/oder physikalische Behandlung der Prostata und gegebenenfalls Blase in Verbindung mit oder nach einer transurethralen Resektion bzw. Vaporisation der Prostata oder in Verbindung mit oder nach einer Bestrahlungstherapie eines Karzinoms der Prostata im Vordergrund stehen. Durch eine gezielte Medikamentenabgabe über die permeabel ausgerüstete Wandung des Prostataballons über einen definierten Zeitraum ist eine lokale Medikamentenapplikation möglich. Die Medikamente werden dabei unmittelbar an der Therapiestelle verabreicht und nicht - im Unterschied zu einer systemischen Therapie - über den gesamten Körper verteilt. Durch die Fokussierung der Medikamentenabgabe in Bezug auf das Zielorgan können geringe Wirkstoffmengen eingesetzt und damit Medikamentenkosten eingespart und unerwünschte systemische Nebenwirkungen vermieden werden.

Weiterer Gegenstand der vorliegenden Erfindung - gemäß einem **zweiten** Aspekt der vorliegenden Erfindung - ist ein sogenanntes Kit (synonym auch als Kit-of-parts oder Set bezeichnet), mit den Merkmalen des Anspruchs 1.

Der Begriff des "Kit", wie er erfindungsgemäß verwendet wird, bezeichnet dabei insbesondere eine Einheit von räumlich getrennten, aber funktional zusammenhängenden, aufeinander abgestimmten bzw. vorgefertigten Komponenten, nämlich Katheter einerseits und Applikationsvorrichtung andererseits.

Ein Vorteil des erfindungsgemäßen Kit ist insbesondere darin zu sehen, dass die beiden aufeinander abgestimmten Komponenten (Katheter einerseits und Applikationsvorrichtung andererseits) jeweils anwendungsfertig vorliegen und vom Endnutzer, insbesondere von einem Arzt oder von medizinischem Personal, ohne Weiteres zusammengeführt bzw. miteinander kombiniert werden können, um den erfindungsgemäßen Katheter einzuführen und das gewünschte Medikament verabreichen zu können. Auf diese Weise wird ein effizienter wie rascher Einsatz ermöglicht, ohne dass die Gefahr von Fehlanwendungen, insbesondere Dosierfehlern oder Verabreichung falscher Medikamente, besteht.

In diesem Zusammenhang haben sich spezielle Wirkstoffkombinationen zur Behandlung der Prostata und gegebenenfalls der Blase besonderes bewährt, wie nachfolgend noch ausgeführt wird.

Weitere Merkmale, Vorteile und Anwendungsmöglichkeiten der vorliegenden Erfindung ergeben sich aus der nachfolgenden Beschreibung von Ausführungsbeispielen anhand der Zeichnungen (Figurendarstellungen) sowie der Zeichnungen (Figurendarstellungen) selbst. Dabei bilden insbesondere alle beschriebenen und/oder bildlich dargestellten Merkmale für sich oder in beliebiger Kombination untereinander den Gegenstand der vorliegenden Erfindung, unabhängig von ihrer Zusammenfassung in Ansprüchen und deren Rückbeziehung.

Es zeigt:
- Fig. 1: eine schematische Darstellung eines erfindungsgemäßen Katheters bzw. eines erfindungsgemäßen Kit im eingeführten bzw. applizierten Zustand gemäß einer bevorzugten Ausführungsform der vorliegenden Erfindung;
- Fig. 2: eine schematische Schnittdarstellung eines erfindungsgemäßen Katheters bzw. eines erfindungsgemäßen Kit gemäß einer bevorzugten Ausführungsform der vorliegenden Erfindung; und
- Fig. 3: eine schematische Darstellung eines erfindungsgemäßen Katheters bzw. eines erfindungsgemäßen Kit mit gefülltem Blasen- und Prostataballon gemäß einer bevorzugten Ausführungsform der vorliegenden Erfindung.

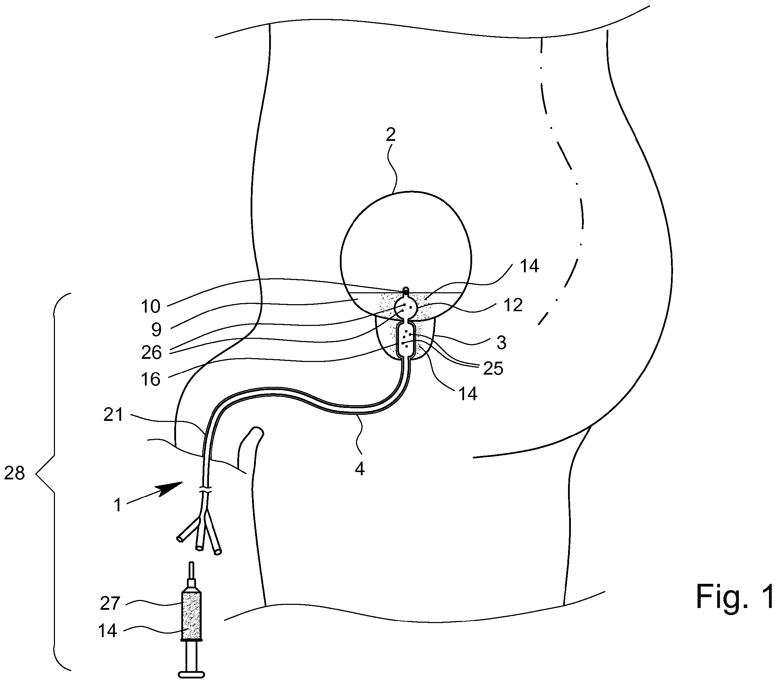

In den Figurendarstellungen gemäß Fig. 1, Fig. 2 und Fig. 3 ist schematisch ein Katheter 1, insbesondere Verweilkatheter, dargestellt, welcher sich bevorzugt zur Behandlung von Funktionsstörungen und/oder Erkrankungen der Prostata 3 und gegebenenfalls der Blase 2, insbesondere zur medikamentösen und/oder physikalischen Behandlung der Prostata 3 und gegebenenfalls der Blase 2, vorzugsweise in Verbindung mit oder nach einer transurethralen Resektion bzw. Vaporisation der Prostata 3 oder in Verbindung mit oder nach einer Bestrahlungstherapie eines Karzinoms der Prostata 3, eignet. Der erfindungsgemäße Katheter 1 weist einen Katheterschlauch 4, einen am Katheterschlauch 4 befindlichen Katheteranschluss 5' und einen befüllbaren und/oder expandierbaren Prostataballon 16 auf. Der Katheterschlauch 4 wiederum weist ein distales Ende 5 und ein proximales Ende 7 auf, wobei sich der Katheteranschluss 5' am distalen Ende 5 befindet. Weiterhin ist ein vom Katheteranschluss 5' bis zu wenigstens einer Ablauföffnung 10 im Bereich des proximalen Endes 7 des Katheterschlauchs 4 reichender erster Kanal 8 vorgesehen. Der Prostataballon 16 befindet sich am proximalen Ende 7 des Katheterschlauchs 4.

Des Weiteren ist ein vom Katheteranschluss 5' bis zu dem Prostataballon 16 im Bereich des proximalen Endes 7 des Katheterschlauchs 4 reichender zweiter Kanal 15 vorgesehen. Der Prostataballon 16 weist eine mittels Poren 25 permeabel ausgebildete Wandung 17 auf, wobei die Größe, insbesondere der Durchmesser, der Poren 25 eingestellt ist derart, dass im eingeführten Zustand des Katheters 1 eine gezielte Abgabe eines Medikaments 14 durch die Wandung 17 hindurch erfolgen kann.

Wie bereits vorstehend ausgeführt, ermöglicht der erfindungsgemäße Katheter auf diese Weise eine effiziente Behandlung von Funktionsstörungen und/oder Erkrankungen der Prostata und gegebenenfalls der Blase, wobei die Behandlung einerseits physikalisch, nämlich mittels Kompression, und andererseits medikamentös, insbesondere durch lokale bzw. topische Medikamentenabgabe, erfolgt.

Durch die mittels Poren permeabel ausgebildete Wandung wird eine optimale und gezielte Abgabe des Medikaments direkt am Therapieort erreicht. Somit kann durch die Poren des Prostataballons das mindestens eine Medikament im Körper des Patienten am Ort der Behandlung, d. h. direkt an der Therapiestelle, zur Verfügung gestellt und gezielt über eine vorgegebene Dauer abgegeben werden. Die Erfindung ermöglicht damit eine Behandlung der Prostata und gegebenenfalls der Blase über ein definiertes Zeitsegment durch die kontinuierliche Abgabe mindestens eines Medikaments über die Poren des Prostataballons unmittelbar an der Therapiestelle, wobei über die Durchlässigkeit der Wandung des Prostataballons die Abgabemenge des Medikaments pro Zeiteinheit exakt gesteuert werden kann.

Wie die Figurendarstellungen gemäß Fig. 1, Fig. 2 und Fig. 3 ferner zeigen, weist die Wandung 17 des Prostataballons 16 eine Mehrzahl von Poren 25 auf. Dabei versteht es sich von selbst, dass es sich anbietet, letztlich nicht nur eine einzige Pore 25 zur Medikamentenzuführung im Bereich der Prostata am proximalen Ende des Katheterschlauchs 4 vorzusehen, sondern im Bereich der betroffenen Region eine Vielzahl von Poren 25 zu realisieren, so dass die Anzahl der Poren 25 in weiten Bereichen variieren kann. Im Allgemeinen weist die Wandung 17 des Prostataballons 16 die Poren 25 in einer Anzahl von 1 bis 1.000 Poren, insbesondere 1 bis 500 Poren, vorzugsweise 2 bis 100 Poren, bevorzugt 2 bis 50 Poren, besonders bevorzugt 2 bis 20 Poren, ganz besonders bevorzugt 3 bis 10 Poren, insbesondere bevorzugt 4 bis 6 Poren, auf.

Wie zuvor bereits ausgeführt, kann die Größe, insbesondere der Durchmesser, der Poren 25 im expandierten Zustand des Prostataballons 16 gleichermaßen in weiten Bereichen variieren. Besonders bewährt hat es sich, wenn die Wandung 17 des Prostataballons 16 im expandierten Zustand Poren 25 mit einer Porengröße, insbesondere mit einem Durchmesser, im Bereich von 1 bis 100 µm, insbesondere 2 bis 80 µm, vorzugsweise 3 bis 60 µm, bevorzugt 4 bis 50 µm, besonders bevorzugt 5 bis 40 µm, ganz besonders bevorzugt 8 bis 30 µm, noch mehr bevorzugt 15 bis 25 µm, aufweist.

Besonders bevorzugt ist es, wenn die Poren 25 durch Laserperforation, insbesondere mittels eines Ultrakurzpulslasers, erzeugt sind. Diesbezüglich kann auf vorstehende Ausführungen verwiesen werden.

Wie zuvor ausgeführt, werden gute Ergebnisse erhalten, wenn die Poren 25 der Wandung 17 des Prostataballons 16 im Rahmen der Herstellung des Prostataballons 16 bzw. im Rahmen der Herstellung der Wandung 17 des Prostataballons 16 erzeugt werden. Bei dieser Ausführungsform werden die Poren 25 der Wandung des Prostataballons 16 bzw. der Wandung 17 des Prostataballons 16 also *in situ* während der Herstellung der Wandung 17, insbesondere während der Herstellung der Wandung 17 mittels Polymerisation und/oder mittels chemischer Modifizierung, erzeugt.

Durch die gezielte Einstellung der Größe der Poren 25 (welche bevorzugt durch Laserperforation und/oder *in-situ*-Generierung während der Herstellung der Wandung 17 erzeugt werden können) und durch die gezielte Auswahl der Porenanzahl kann die Abgabe eines Medikaments 14 durch die Wandung 17 des Prostataballons 16 gezielt gesteuert werden. Die Freisetzung des Medikaments 14 erfolgt somit unmittelbar am gewünschten Therapieort.

Insbesondere ist es erfindungsgemäß vorgesehen, dass die Wandung 17 des Prostataballons 16 ausgebildet ist derart, insbesondere die Anzahl und/oder Größe der Poren 25 eingestellt ist derart, dass im eingeführten und medikamentenbefüllten Zustand des Katheters 1 die Abgabe des Medikaments 14, insbesondere durch Diffusion, über einen Zeitraum von mehr als 1 Stunde, vorzugsweise von mehr als 8 Stunden, insbesondere im Bereich von 18 bis 60 Stunden, bevorzugt im Bereich von 24 bis 48 Stunden, erfolgt.

Neben der gezielt ausgebildeten Permeabilität der Wandung 17 des Prostataballons 16 kann die Freisetzung des Medikaments 14 auch zusätzlich durch die Zusammensetzung des freizusetzenden Medikaments 14 gezielt gesteuert werden, insbesondere durch deren Dichte und Viskosität sowie durch die Molekülgröße bzw. das Molekulargewicht der eingesetzten Wirkstoffe etc.

Was das Material des Prostataballons 16 anbelangt, so ist grundsätzlich jedes Material geeignet, welches physiologisch unbedenklich ist, für diese Art von medizinischer Anwendung geeignet ist, insbesondere eine gewisse Druckresistenz aufweist, und kompatibel in Bezug auf das zu verabreichende Medikament ausgebildet ist.

Insbesondere kann der Prostataballon 16 mindestens ein vorzugsweise organisches Polymer aufweisen und/oder zumindest im Wesentlichen hieraus bestehen. Mit anderen Worten kann der Prostataballon 16 gemäß dieser Ausführungsform auf Basis mindestens eines vorzugsweise organischen Polymers ausgebildet sein.

Gemäß einer besonderen Ausführungsform weist der Prostataballon 16 (i) Kautschuk, insbesondere Naturkautschuk, Isoprenkautschuk, Polyurethankautschuk, Acrylnitril-Butadien-Kautschuk und/oder Styrol-Butadien-Kautschuk; (ii) Polyurethane, insbesondere thermoplastische Polyurethane; (iii) Polyvinylchlorid (PVC); (iv) Latex und/oder (v) Silikon; vorzugsweise Silikon, auf und/oder besteht zumindest im Wesentlichen hieraus.

Die Stärke bzw. Dicke der Wandung 17 des Prostataballons 16 kann gleichermaßen in weiten Bereichen variieren. Bevorzugt ist es, wenn die Wandung 17 des Prostataballons 16 im nichtexpandierten Zustand eine Stärke, insbesondere Dicke, im Bereich von 50 bis 500 µm, insbesondere 100 bis 450 µm, vorzugsweise 150 bis 400 µm, bevorzugt 200 bis 300 µm, aufweist und/oder wenn die Wandung 17 des Prostataballons 16 im expandierten Zustand eine Stärke, insbesondere Dicke, im Bereich von 10 bis 250 µm, insbesondere 20 bis 200 µm, vorzugsweise 30 bis 150 µm, bevorzugt 40 bis 100 µm, aufweist.

Was die genaue Positionierung des Prostataballons anbelangt, so ist es erfindungsgemäß bevorzugt, wenn der Prostataballon 16 angeordnet ist derart, insbesondere der Prostataballon 16 am proximalen Ende 7 des Katheterschlauchs 4 positioniert ist derart, dass er sich im eingeführten Zustand des Katheters 1 im Bereich der Prostata 3 befindet (vgl. Fig. 1 mit einer Darstellung des erfindungsgemäßen Katheters 1 im eingeführten Zustand).

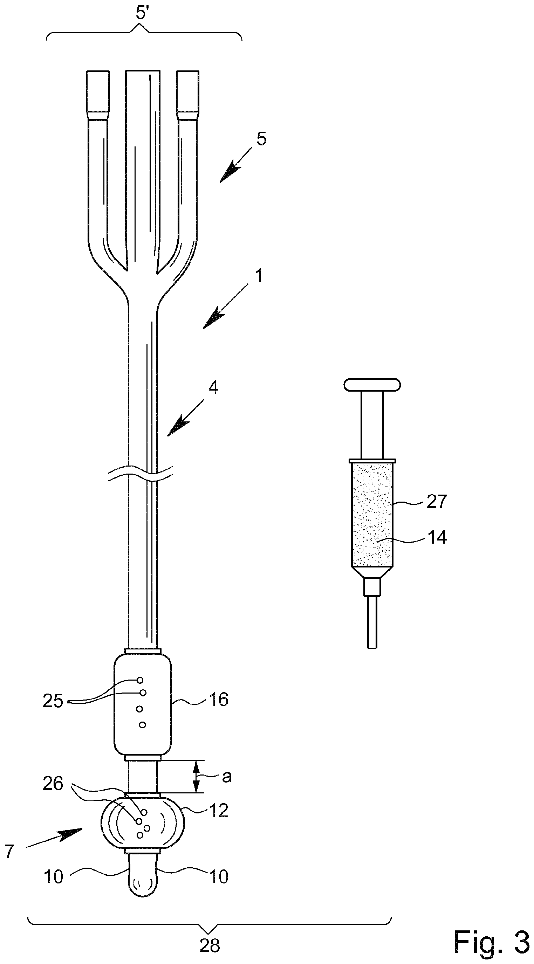

Wie den Figurendarstellungen, insbesondere Fig. 1 und Fig. 3, zu entnehmen ist, ist der Prostataballon 16 nach Befüllung und/oder im expandierten Zustand zumindest im Wesentlichen zylinderförmig ausgebildet. Die zylinderförmige Ausgestaltung stellt sicher, dass es zu einer gleichmäßigen Aufweitung der Harnröhre im Bereich der Prostata kommt.

Weiterhin weist der Prostataballon 16, insbesondere in Abhängigkeit von der Größe der zu behandelnden Prostata 3 und/oder der ausrezesierten Prostataloge, eine Länge im Bereich zwischen 2 cm und 6 cm auf.

Des Weiteren weist der Prostataballon 16 im expandierten bzw. (maximal) befüllten Zustand einen äußeren Durchmesser im Bereich von 20 bis 30 Charr., vorzugsweise 22 Charr. bis 26 Charr., insbesondere von 24 Charr., auf.

Das Volumen des Prostataballons 16 im expandierten Zustand kann gleichermaßen in weiten Bereichen variieren: Im Allgemeinen weist der Prostataballon 16 im expandierten Zustand ein Volumen im Bereich von 10 bis 150 ml, insbesondere 15 bis 120 ml, vorzugsweise 20 bis 100 ml, auf. Insbesondere in Abhängigkeit von der ausrezesierten Prostataloge können beispielsweise Prostataballone 16 mit drei unterschiedlichen Größen, vorzugsweise mit in einer Bandbreite des Volumens von 40 bis 100 ml, vorgesehen sein: Gemäß einer ersten Ausführungsform kann der Prostataballon 16 im expandierten Zustand ein Volumen im Bereich von 10 bis 50 ml, insbesondere 15 bis 45 ml, vorzugsweise 20 bis 40 ml, aufweisen. Gemäß einer zweiten, hierzu alternativen Ausführungsform kann der Prostataballon 16 im expandierten Zustand ein Volumen im Bereich von 40 bis 80 ml, insbesondere 45 bis 75 ml, vorzugsweise 40 bis 70 ml, aufweisen. Gemäß einer dritten, wiederum alternativen Ausführungsform kann der Prostataballon 16 im expandierten Zustand ein Volumen im Bereich von 70 bis 120 ml, insbesondere 75 bis 110 ml, vorzugsweise 80 bis 100 ml, aufweisen. Bei Versuchen ist festgestellt worden, dass bei den vorgenannten Volumina bzw. Größen des Prostataballons 16 eine hinreichende, aber gleichzeitig die Prostata 3 nicht schädigende Kompression sowie eine die Harnröhre 21 nicht schädigende Aufweitung erfolgt.

Aufgrund der Medikamentenabgabe über die Wandung 17 des Prostataballons 16 ergibt sich über die Abgabezeit eine Verringerung des Lumens des Prostataballons 16. Erfindungsgemäß ist es deshalb vorgesehen, dass die Wandung 17 des Prostataballons 16 ausgebildet ist derart, insbesondere die Anzahl und/oder Größe der Poren 25 eingestellt ist derart, dass nach Medikamentenabgabe im noch befüllten Zustand ein Außendurchmesser des Prostataballons 16 von 20 Charr. (Charriere) nicht unterschritten wird. Dadurch, dass sich durch die Medikamentenabgabe der Außendurchmesser auf einen Wert von maximal 20 Charr. verringert, ist sichergestellt, dass der Prostataballon 16 selbst nach Medikamentenabgabe noch eine hinreichende Kompression der Prostata 3 gewährleistet.

Im Rahmen des erfindungsgemäßen Katheters ist es weiterhin vorgesehen, dass der Prostataballon 16 mit mindestens einem Medikament 14, insbesondere in flüssiger oder fließfähiger Form, vorzugsweise in Form einer mindestens einen pharmazeutischen Wirkstoff enthaltenden flüssigen Zusammensetzung, befüllbar ausgebildet ist und/oder befüllt ist (letzteres im applizierten Zustand). Das Medikament 14 kann dabei in viskoser und/oder gelförmiger Form ausgebildet sein und/oder in Form eines Gels vorliegen. Gemäß einer bevorzugten Ausführungsform kann das Medikament 14 bei einer Temperatur von 25 °C eine Brookfield-Viskosität im Bereich von 500 bis 100.000 mPas, insbesondere 750 bis 10.000 mPas, bevorzugt 1.000 bis 7.500 mPas, besonders bevorzugt 1.250 bis 5.000 mPas, ganz besonders bevorzugt 1.300 bis 3.000 mPas, aufweisen. Des Weiteren kann das Medikament 14 bei einer Temperatur von 25 °C und bei Atmosphärendruck (1.013 hPa) eine Dichte im Bereich von 0,7 bis 1,5 g/cm³, insbesondere 0,8 bis 1,4 g/cm³, bevorzugt 0,85 bis 1,3 g/cm³, besonders bevorzugt 0,9 bis 1,2 g/cm³, ganz besonders bevorzugt 0,95 bis 1,1 g/cm³, aufweisen. Insbesondere wird das Medikament 14 bzw. der im Medikament 14 enthaltene pharmazeutische Wirkstoff in Bezug auf die Porengröße und/oder Porenanzahl der Wandung 17 des Prostataballons 16 abgestimmt, insbesondere um eine ideale Freisetzung des Medikaments 14 für die lokale Therapie zu ermöglichen.

Zusätzlich kann es vorgesehen sein, dass der Prostataballon 16 außerdem beschichtet ist, insbesondere mit mindestens einem Medikament 14, und/oder dass auf den Prostataballon 16 außerdem mindestens ein Medikament 14 aufgebracht ist. Alternativ kann es gemäß dieser Ausführungsform aber auch vorgesehen sein, dass das mindestens eine Medikament 14 in die Wandung 17 des Prostataballons 16 eingelagert und/oder eingebracht ist und/oder an die Wandung 17 des Prostataballons 16 angelagert und/oder angebunden und/oder hiermit verbunden ist.

Was den Katheter 1 bzw. den Katheterschlauch 4 anbelangt, so weist der Katheter 1, insbesondere der Katheterschlauch 4, einen äußeren Durchmesser im Bereich von 12 bis 30 Charr., insbesondere 14 bis 28 Charr., vorzugsweise 16 bis 26 Charr., bevorzugt 18 bis 24 Charr., auf.

Weiterhin weist der Katheterschlauch 4 einen äußeren Mantel 6, insbesondere auf Basis von Silikon, auf. Die Gesamtlänge des Katheters 1 kann in weiten Bereichen variieren. Üblicherweise weist der Katheter 1 eine Gesamtlänge im Bereich von 10 bis 100 cm, insbesondere 15 bis 50 cm, vorzugsweise 20 bis 40 cm, auf.

Durch die vorgenannten Abmessungen wird eine optimale Harnableitung einerseits und eine effiziente therapeutische Behandlung andererseits ermöglicht.

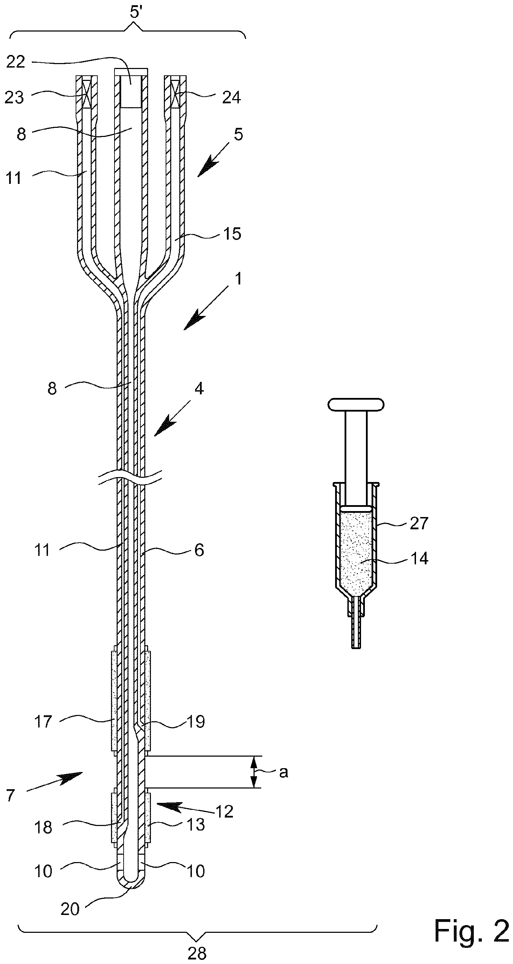

Wie sich den Figurendarstellungen gemäß Fig. 2 und Fig. 3 weiterhin entnehmen lässt, ist am proximalen Ende 7 des Katheterschlauchs 4 eine endseitig geschlossene Spitze 20 vorgesehen; dabei kann das Ende der Spitze 20 vorzugsweise abgerundet ausgebildet sein. Weiterhin kann zur besseren Handhabung die Spitze 20 gebogen ausgebildet sein. Die Spitze 20 kann insbesondere mindestens eine Ablauföffnung 10, vorzugsweise mindestens zwei Ablauföffnungen 10, insbesondere zum Ablass von Urin 9 aus der Blase 2, aufweisen.

Die Ablauföffnung 10 kann grundsätzlich kreis- oder schlitzförmig ausgebildet sein, wobei eine schlitzförmige Ausbildung bevorzugt ist, insbesondere zum besseren Ablass von Urin 9.

Bevorzugt ist eine Mehrzahl von Ablauföffnungen 10 vorgesehen; insbesondere sind mindestens zwei Ablassöffnungen 10 vorgesehen.

Die Ablauföffnung(en) 10 und der erste Kanal 8 dienen, wie zuvor erläutert, zum Ablass von Urin 9 aus der Blase 2.

Wie die Figurendarstellungen gemäß Fig. 1, Fig. 2 und Fig. 3 weiterhin zeigen, sind im Bereich des proximalen Endes 7 des Katheterschlauchs 4 Mittel 12 zur Positionierung des proximalen Endes 7, vorzugsweise zur insbesondere dauerhaften Fixierung des proximalen Endes 7 während der Verweildauer des Katheters 1, vorgesehen. Insbesondere ist als Mittel 12 zur Positionierung des proximalen Endes 7 ein Blasenballon 12, vorzugsweise ein expandierbarer und/oder befüllbarer, insbesondere mit einem Medikament 14 befüllbarer, Blasenballon 12, vorgesehen.

Grundsätzlich ist der Prostataballon 16 allein bereits ausreichend, um eine ausreichende Haltefunktion des Katheters 1 zu gewährleisten. Durch das Vorsehen eines zusätzlichen, optional vorhandenen Blasenballons 12 lässt sich diese Haltefunktion aber noch verbessern. Zudem bietet der optionale Blasenballon 12 gegebenenfalls die Möglichkeit, auch die Blase 2 mitzutherapieren, wie nachfolgend im Detail ausgeführt.

Im Allgemeinen ist der Blasenballon 12 im expandierten Zustand und/oder nach Befüllung zumindest im Wesentlichen kugelförmig ausgebildet. Durch die Kugelform kann die Anschlagfunktion des Blasenballons 12 im eingeführten Zustand in einfacher Weise sichergestellt werden.

Weiterhin ist ein vom Katheteranschluss 5' bis zu dem befüllbaren und/oder expandierbaren Blasenballon 12, insbesondere in den Bereich des proximalen Endes 7 des Katheterschlauchs 4, reichender dritter Kanal 11 vorgesehen. Hierüber erfolgt die Expansion des Blasenballons 12 und gegebenenfalls auch eine Medikamentenzufuhr in den Blasenballon 12.

Um eine optimale Positionierung und auf diese Weise eine optimale Therapie zu ermöglichen, ist es vorgesehen, dass der lichte Abstand a des Prostataballons 16 zum Blasenballon 12 wenigstens 0,5 cm und vorzugsweise 1,0 cm bis 3,0 cm, insbesondere etwa 1,5 cm, beträgt. Durch Anordnung des Prostataballons 16 in einem genau vorgegebenen Abstand zum Blasenballon 12 wird die Medikamentenzufuhr in der zu behandelnden Prostataregion gewährleistet.

Wie der Prostataballon 16, so kann auch der Blasenballon 12 grundsätzlich durch beliebige Materialien ausgebildet sein, welche physiologisch unbedenklich sind, unter Anwendungsbedingungen geeignet sind, insbesondere eine Druckresistenz aufweisen, und zudem eine medizinische Anwendung ermöglichen, insbesondere kompatibel in Bezug auf ein zu verabreichendes Medikament sind.

Insbesondere kann der Blasenballon 12 mindestens ein vorzugsweise organisches Polymer aufweisen und/oder zumindest im Wesentlichen hieraus bestehen; mit anderen Worten kann der Blasenballon 12 gemäß dieser Ausführungsform auf Basis mindestens eines vorzugsweise organischen Polymers ausgebildet sein.

Insbesondere kann es vorgesehen sein, dass der Blasenballon 12 (i) Kautschuk, insbesondere Naturkautschuk, Isoprenkautschuk, Polyurethankautschuk, Acrylnitril-Butadien-Kautschuk und/oder Styrol-Butadien-Kautschuk; (ii) Polyurethane, insbesondere thermoplastische Polyurethane; (iii) Polyvinylchlorid (PVC); (iv) Latex und/oder (v) Silikon; vorzugsweise Silikon, aufweist und/oder zumindest im Wesentlichen hieraus besteht.

Wie im Fall des Prostataballons 16, so ist auch im Fall des Blasenballons 12 das Volumen im expandierten Zustand variabel: Insbesondere kann es vorgesehen sein, dass der Blasenballon 12 im expandierten Zustand ein Volumen im Bereich von 10 bis 200 ml, insbesondere 15 bis 150 ml, vorzugsweise 20 bis 100 ml, bevorzugt 25 bis 75 ml, aufweist.

Wie im Fall des Prostataballons 16, so ist auch im Fall des Blasenballons 12 die Größe des Blasenballons 12 im expandierten Zustand variabel: Im Allgemeinen weist der Blasenballon 12 im expandierten bzw. (maximal) befüllten Zustand einen äußeren Durchmesser im Bereich von 20 bis 30 Charr., vorzugsweise 22 Charr. bis 26 Charr., insbesondere von 24 Charr., auf.

Auch die Stärke bzw. Dicke des Blasenballons 12 kann in bestimmten Bereichen variieren. Es kann vorgesehen sein, dass die Wandung 13 des Blasenballons 12 im nichtexpandierten Zustand eine Stärke, insbesondere Dicke, im Bereich von 50 bis 500 µm, insbesondere 100 bis 450 µm, vorzugsweise 150 bis 400 µm, bevorzugt 200 bis 300 µm, aufweist und/oder dass die Wandung 13 des Blasenballons 12 im expandierten Zustand eine Stärke, insbesondere Dicke, im Bereich von 10 bis 250 µm, insbesondere 20 bis 200 µm, vorzugsweise 30 bis 150 µm, bevorzugt 40 bis 100 µm, aufweist.

Wie sich den Figurendarstellungen gemäß Fig. 1, Fig. 2 und Fig. 3 entnehmen lässt, weist der Blasenballon 12 eine Wandung 13 auf bzw. wird der Blasenballon 12 von einer Wandung 13 begrenzt. Grundsätzlich kann dabei die Wandung 13 entweder impermeabel, insbesondere medikamentenundurchlässig, ausgebildet sein oder aber - gemäß einer alternativen, erfindungsgemäß bevorzugten Ausführungsform - permeabel, insbesondere medikamentendurchlässig, ausgebildet sein. Im Fall der permeablen Ausbildung der Wandung 13 des Blasenballons 12 ist - wie im Fall des Prostataballons 16 - eine gezielte Medikamentenabgabe möglich, so dass auf diese Weise auch eine zeitgleiche Therapie der Blase 2 ermöglicht wird.

Im Rahmen der vorliegenden Erfindung hat sich eine zeitgleiche Therapie der Blase 2 durch eine gezielte Medikamentenabgabe mittels permeabler Ausbildung der Wandung 13 des Blasenballons 12 als besonders effizient erwiesen: Bei der Behandlungsstrategie der Erkrankung der Prostata 3 bzw. der Blase 2 kann insbesondere davon ausgegangen werden, dass die Prostata 3 und die Blase 2 eine anatomische, funktionelle und physiologische Einheit bilden. Dies bedingt auch bei funktionellen und pathophysiologischen Störungen und Erkrankungen, dass sowohl eine Behandlung der Prostata als auch der Blase empfehlenswert ist. Insbesondere entsteht bei der TUR-P ein Symptomenkomplex aus Dysurie, Pollakisurie und starkem Dranggefühl, da man durch die Resektion des Prostatagewebes eine große Wundfläche schafft, die diesen Symptomenkomplex verursacht; wenn sich nun die Blase 2 mit Flüssigkeit bzw. Urin füllt oder ein transurethraler Katheter in die Blase 2 bzw. Prostataloge positioniert wird, vergrößert bzw. verstärkt sich dieser Reiz, und reflektorisch versucht daher die Blase 2, durch die Kontraktion den Inhalt (also den Urin) zu entleeren bzw. den Katheter 1 aus der Blase 2 "herauszudrücken", so dass sehr schmerzhafte Blasentenesmen, d.h. Blasenkrämpfe, entstehen, welche für den Patienten äußerst unangenehm sind und zu Komplikationen führen können. Daher ist es vorteilhaft, wenn auch über den Blasenballon 12 eine gezielte Medikamentenabgabe mittels permeabler Ausbildung der Wandung 13 des Blasenballons 12 erfolgen kann, so dass derartige Blasentenesmen reduziert bzw. aufgehoben werden können. Auch kommt es im Rahmen der Vaporisation der Prostata 3 oder in Verbindung mit einer Bestrahlungstherapie eines Prostatakarzinoms oftmals zu einer unerwünschten Schädigung der Blase 2, welche über einen permeablen Blasenballon 12 ohne Weiteres therapiert werden kann.

Erfindungsgemäß bevorzugt ist es daher, wenn der Blasenballon 12 eine permeabel, mittels Poren 26 permeabel, ausgebildete Wandung 13 aufweist, insbesondere derart, dass die Größe, insbesondere der Durchmesser, der Poren 26 eingestellt ist derart, dass im eingeführten Zustand des Katheters 1 eine gezielte Medikamentenabgabe durch die Wandung 13 hindurch erfolgen kann. In Bezug auf die permeable Ausbildung der Wandung 13 des Blasenballons 12 gelten alle Ausführungen zu der permeablen Ausbildung der Wandung 17 des Prostataballons 16 entsprechend.

Im Fall der permeablen Ausbildung der Wandung 13 des Blasenballons 12 kann die Wandung 13 des Blasenballons 12 eine Mehrzahl von Poren 26 aufweisen. Insbesondere kann die Wandung 13 des Blasenballons 12 dabei 1 bis 1.000 Poren, insbesondere 1 bis 500 Poren, vorzugsweise 2 bis 100 Poren, bevorzugt 2 bis 50 Poren, besonders bevorzugt 2 bis 20 Poren, ganz besonders bevorzugt 3 bis 10 Poren, insbesondere bevorzugt 4 bis 6 Poren, aufweisen. Insbesondere zur effizienten Medikamentenabgabe sollte dabei die Wandung 13 des Blasenballons 12 im expandierten Zustand Poren 26 mit einer Porengröße, insbesondere mit einem Durchmesser, im Bereich von 1 bis 100 µm, insbesondere 2 bis 80 µm, vorzugsweise 3 bis 60 µm, bevorzugt 4 bis 50 µm, besonders bevorzugt 5 bis 40 µm, ganz besonders bevorzugt 8 bis 30 µm, noch mehr bevorzugt 15 bis 25 µm, aufweisen. Wie im Fall der Poren 25 des Prostataballons 16 ist es erfindungsgemäß bevorzugt, wenn die Poren 26 des Blasenballons 12 (d. h. also die Poren 26 der Wandung 13 des Blasenballons 12) durch Laserperforation, insbesondere mittels eines Ultrakurzpulslasers, erzeugt sind. Wie im Fall der Poren 25 des Prostataballons 16, so können alternativ aber gleichermaßen besonders gute Ergebnisse erhalten werden, wenn die Poren 26 des Blasenballons 12 (d. h. also die Poren 26 der Wandung 13 des Blasenballons 12) im Rahmen der Herstellung des Blasenballons 12 bzw. im Rahmen der Herstellung der Wandung 13 des Blasenballons 12 erzeugt werden. Zur Vermeidung unnötiger Wiederholungen kann daher auf obige Ausführungen zum Prostataballon 16 verwiesen werden, welche in diesem Zusammenhang entsprechend gelten.

Im Fall der permeablen Ausbildung der Wandung 13 des Blasenballons 12 ist es erfindungsgemäß bevorzugt, wenn die Wandung 13 des Blasenballons 12 ausgebildet ist derart, insbesondere die Anzahl und/oder Größe der Poren 26 eingestellt ist derart, dass im eingeführten und medikamentenbefüllten Zustand des Katheters 1 die Abgabe des Medikaments 14, insbesondere durch Diffusion, über einen Zeitraum von mehr als 1 Stunde, vorzugsweise von mehr als 8 Stunden, insbesondere im Bereich von 18 bis 60 Stunden, bevorzugt im Bereich von 24 bis 48 Stunden, erfolgt.

Im Fall der permeabel ausgebildeten Wandung 13 des Blasenballons 12 ist es erfindungsgemäß bevorzugt vorgesehen, dass die Wandung 13 des Blasenballons 12 ausgebildet ist derart, insbesondere die Anzahl und/oder Größe der Poren 26 eingestellt ist derart, dass nach Medikamentenabgabe im noch befüllten Zustand ein Außendurchmesser des Blasenballons 12 von 20 Charr. (Charriere) nicht unterschritten wird. Durch die Verringerung des Außendurchmessers auf einen Wert von maximal 20 Charr. ist sichergestellt, dass sich der Blasenballon 12 nicht aus der Blase 2 herausziehen lässt.

Im Fall eines permeabel ausgebildeten Blasenballons 12 ist es erfindungsgemäß vorgesehen, dass der Blasenballon 12 mit mindestens einem Medikament 14, insbesondere in flüssiger oder fließfähiger Form, vorzugsweise in Form einer mindestens einen pharmazeutischen Wirkstoff enthaltenden flüssigen Zusammensetzung, befüllbar ausgebildet ist und/oder befüllt ist (letzteres im applizierten Zustand). Das Medikament 14 kann in viskoser und/oder gelförmiger Form ausgebildet sein und/oder in Form eines Gels vorliegen. Gemäß einer bevorzugten Ausführungsform kann das Medikament 14 bei einer Temperatur von 25 °C eine Brookfield-Viskosität im Bereich von 500 bis 100.000 mPas, insbesondere 750 bis 10.000 mPas, bevorzugt 1.000 bis 7.500 mPas, besonders bevorzugt 1.250 bis 5.000 mPas, ganz besonders bevorzugt 1.300 bis 3.000 mPas, aufweisen. Weiterhin kann es vorgesehen sein, dass das Medikament 14 bei einer Temperatur von 25 °C und bei Atmosphärendruck (1.013 hPa) eine Dichte im Bereich von 0,7 bis 1,5 g/cm³, insbesondere 0,8 bis 1,4 g/cm³, bevorzugt 0,85 bis 1,3 g/cm³, besonders bevorzugt 0,9 bis 1,2 g/cm³, ganz besonders bevorzugt 0,95 bis 1,1 g/cm³, aufweist.

Wie die Figurendarstellungen gemäß Fig. 2 und Fig. 3 zeigen, weist der Katheteranschluss 5' mindestens zwei, vorzugsweise mindestens drei, insbesondere drei, Anschlussenden am distalen Ende 5 auf, insbesondere ein Anschlussende des ersten Kanals 8, ein Anschlussende des zweiten Kanals 15 und ein Anschlussende des dritten Kanals 11.

Erfindungsgemäß ist es bevorzugterweise vorgesehen, dass der erste, zweite und dritte Kanal 8, 15, 11 mit einem Verschlusselement 22, 24, 23, insbesondere in Form eines Stopfens, Stöpsels, Ventils oder dergleichen, jeweils verschließbar ausgebildet sind. Dabei kann der erste Kanal 8 mit einem Stöpsel 22, gegebenenfalls mit Ventilmechanismus, verschließbar ausgebildet sein, während der zweite und dritte Kanal 15, 11 jeweils mit einem Ventil 24, 23 verschließbar ausgebildet sein können.

Wie sich aus Fig. 2 ergibt, ist der zweite Kanal 15 über eine Auslassöffnung 19 mit dem Prostataballon 16 verbunden bzw. mündet hierin. Der Prostataballon 16 ist über eine Auslassöffnung 19 im zweiten Kanal 15 expandierbar und/oder befüllbar. Insbesondere ist die Wandung 17 des Prostataballons 16 an den äußeren Rändern mit dem Katheterschlauch 4, insbesondere mit dem Mantel 6, umlaufend verbunden und bildet auf diese Weise einen Ringraum aus, in welchen eine Auslassöffnung 19 des zweiten Kanals 15 mündet.

Der dritte Kanal 11 wiederum ist über eine Auslassöffnung 18 mit dem Blasenballon 12 verbunden bzw. mündet hier. Weiterhin ist der Blasenballon 12 über die Auslassöffnung 18 im dritten Kanal 11 expandierbar und/oder befüllbar. Insbesondere ist die Wandung 13 des Blasenballons 12 an den äußeren Rändern mit dem Katheterschlauch 4, insbesondere mit dem Mantel 6, umlaufend verbunden und bildet auf diese Weise einen Ringraum aus, in welchen eine Auslassöffnung 18 des dritten Kanals 11 mündet.

Im befüllten Zustand hat der erfindungsgemäße Katheter insbesondere folgende Funktionen: Durch die Befüllung bzw. Expansion des Prostataballons wird die Prostata komprimiert und ein bestehendes Ödem herausgepresst bzw. komprimiert (physikalischer Ansatz). Es erfolgt weiterhin eine lokale bzw. topisch begrenzte medikamentöse Therapie der Prostata, welche durch Abräumvorgänge devitalisierter Zellen entzündlich verändert ist oder eine große Wundfläche insbesondere infolge der transurethralen Resektion der Prostata (TUR-P) aufweist; mittels Diffusion aus der permeablen Wandung des Prostataballons können geeignete Wirkstoffe unmittelbar am Therapieort kontrolliert abgegeben werden (medikamentöser Ansatz). Für den Fall, dass zusätzlich auch eine permeable Wandung des Blasenballons vorgesehen ist, kann auch eine lokale bzw. topisch begrenzte medikamentöse Therapie der Blase in entsprechender Weise vorgenommen werden (z. B. lokale Therapie einer infolge einer Bestrahlung entstandenen Urozystitis).

Wie sich den Figurendarstellungen gemäß Fig. 1, Fig. 2 und Fig. 3 gleichermaßen entnehmen lässt, ist weiterer Gegenstand der vorliegenden Erfindung ein Kit 28 (synonym auch als Kit-of-parts oder Set bezeichnet), in Form eines Kathetersystems, welches sich bevorzugt zur Behandlung von Funktionsstörungen und/oder Erkrankungen der Prostata 3 und gegebenenfalls der Blase 2, insbesondere zur medikamentösen und/oder physikalischen Behandlung der Prostata 3 und gegebenenfalls der Blase 2, vorzugsweise in Verbindung mit oder nach einer transurethralen Resektion und/oder Vaporisation der Prostata 3 oder in Verbindung mit oder nach einer Bestrahlungstherapie eines Karzinoms der Prostata 3, eignet, wobei das Kit 28 einerseits einen wie zuvor beschrieben Katheter 1 nach der vorliegenden Erfindung und andererseits mindestens eine mindestens ein Medikament 14 enthaltende Applikationsvorrichtung 27 zur Verabreichung des Medikaments 14, insbesondere in Form einer vorzugsweise sterilen Spritze, bevorzugt Kolbenspritze, insbesondere zur Befüllung des Katheters 1, insbesondere des Prostataballons 16 und gegebenenfalls des Blasenballons 12, mit dem Medikament 14, umfasst.

Auf diese Weise wird ein anwendungsfertiges Kit 28 aus räumlich getrennten, aber funktional zusammenhängenden Komponenten (nämlich dem Katheter 1 einerseits und der Applikationsvorrichtung 27 andererseits) bereitgestellt, so dass eine unmittelbare und zeitnahe Anwendung unter Vermeidung von Anwendungsfehlern, insbesondere Dosierfehlern, erfolgen kann. Für weitergehende diesbezügliche Ausführungen kann auf die vorstehenden Ausführungen in Bezug auf das erfindungsgemäße Kit verwiesen werden.

Besonders gute Therapieeffekte lassen sich erzielen, wenn das Medikament 14 mindestens einen pharmazeutischen Wirkstoff, insbesondere eine Kombination von mindestens zwei, vorzugsweise mindestens drei, voneinander verschiedenen pharmazeutischen Wirkstoffen enthält,
wobei der pharmazeutische Wirkstoff ausgewählt ist aus der Gruppe von
(i) Antiphlogistika, insbesondere ausgewählt aus Serrapeptase, Allantoin, Coxibe, Acetylsalicylsäure, Ibuprofen, Kortikosteroiden, insbesondere Hydrocortison, Prednisolon, Methylprednisolon, Budesonid und/oder Dexamethason, und Diclofenac sowie deren Mischungen und Kombinationen, vorzugsweise Allantoin;
(ii) Antiödematika, insbesondere ausgewählt aus Bromelain und Aescin sowie deren Mischungen und Kombinationen;
(iii) Spasmolytika, insbesondere ausgewählt aus Butylscopolamin (Buscopan), Hyoscyamin, Scopolamin und Ipratropium sowie deren Mischungen und Kombinationen;
(iv) Anticholinergika, insbesondere ausgewählt aus Trospiumchlorid, Tolterodin, Darifenacin, Solifenacin, Oxybutynin und Propiverin sowie deren Mischungen und Kombinationen, vorzugsweise Trospiumchlorid;
(v) Wundheilmitteln, insbesondere ausgewählt aus Dexpanthenol und dessen Estern, Chondroitin und dessen Salzen, insbesondere Chondroitinsulfat, Zwiebelextrakt, Beinwellextrakt, Hyaluronsäure und deren Salzen, Bisabolol, Ringelblumenextrakt, Kamilleextrakt und Hamamelisextrakt sowie deren Mischungen und Kombinationen, vorzugsweise Dexpanthenol, Chondroitin und dessen Salzen und Hyaluronsäure und deren Salzen;
(vi) Antiseptika, insbesondere ausgewählt aus Chlorhexidin, Octenidin und Polyhexanid sowie deren Mischungen und Kombinationen, vorzugsweise Polyhexanid;
(vii) Lokalanästhetika, insbesondere Lidocain,
sowie deren Mischungen und Kombinationen.

Besonders gute Therapieerfolge werden erzielt, wenn das Medikament 14 mindestens einen pharmazeutischen Wirkstoff, insbesondere eine Kombination von mindestens zwei, vorzugsweise mindestens drei, voneinander verschiedenen pharmazeutischen Wirkstoffen, enthält, wobei der pharmazeutische Wirkstoff ausgewählt ist aus der Gruppe von Antiphlogistika (Antiinflammatorika), Antiödematika und Wundheilmitteln sowie deren Mischungen und Kombinationen und insbesondere eine Kombination mindestens eines Antiphlogistikums und mindestens eines Wundheilmittels umfasst.

Gemäß einer bevorzugten Ausführungsform der vorliegenden Erfindung ist es vorgesehen, dass das Medikament 14 einen Gehalt an Wirkstoff(en) im Bereich von 0,00001 bis 80 Gew.-%, insbesondere 0,0001 bis 50 Gew.-%, vorzugsweise 0,001 bis 25 Gew.-%, bevorzugt 0,005 bis 20 Gew.-%, besonders bevorzugt 0,01 bis 10 Gew.-%, bezogen auf das Medikament 14, aufweist und/oder dass das Medikament 14 neben dem Wirkstoff einen pharmazeutisch kompatiblen Exzipienten, insbesondere Wasser oder eine vorzugsweise isotonische wässrige Kochsalzlösung, vorzugsweise Ringerlösung, enthält, insbesondere in Mengen im Bereich von 20 bis 99,99999 Gew.-%, insbesondere 50 bis 99,9999 Gew.-%, vorzugsweise 75 bis 99,999 Gew.-%, bevorzugt 80 bis 99,995 Gew.-%, besonders bevorzugt 90 bis 99,99 Gew.-%, bezogen auf das Medikament 14.

Besonders gute Therapieerfolge werden erzielt, wenn das Medikament 14 eine Kombination von mindestens zwei, vorzugsweise mindestens drei, voneinander verschiedenen pharmazeutischen Wirkstoffen enthält, wobei die Kombination mindestens ein Wundheilmittel zusammen mit mindestens einem Antiseptikum und/oder mindestens einem Antiphlogistikum (Antiinflammatorikum) umfasst, vorzugsweise Dexpanthenol zusammen mit Polyhexanid und/oder Allantoin, besonders bevorzugt Dexpanthenol zusammen mit Polyhexanid und gegebenenfalls Allantoin. Die Mengen an Wundheilmittel, Antiseptikum und Antiphlogistikum (Antiinflammatorikum), können dabei in weiteren Grenzen variieren. Insbesondere kann das Medikament 14, bezogen auf das Medikament 14, Wundheilmittel, insbesondere Dexpanthenol, in Mengen von 0,001 bis 50 Gew.-%, insbesondere 0,01 bis 30 Gew.-%, vorzugsweise 0,1 bis 20 Gew.-%, bevorzugt 0,5 bis 10 Gew.-%, besonders bevorzugt 1 bis 6 Gew.-%, enthalten. Weiterhin kann das Medikament 14, bezogen auf das Medikament 14, Antiseptikum, insbesondere Polyhexanid, in Mengen von 0,0001 bis 20 Gew.-%, insbesondere 0,001 bis 10 Gew.-%, vorzugsweise 0,01 bis 5 Gew.-%, bevorzugt 0,05 bis 2 Gew.-%, besonders bevorzugt 0,05 bis 1 Gew.-%, enthalten. Des Weiteren kann das Medikament 14, bezogen auf das Medikament 14, Antiphlogistikum (Antiinflammatorikum), insbesondere Allantoin, in Mengen von 0,0001 bis 20 Gew.-%, insbesondere 0,001 bis 10 Gew.-%, vorzugsweise 0,01 bis 5 Gew.-%, bevorzugt 0,05 bis 2 Gew.-%, besonders bevorzugt 0,05 bis 1 Gew.-%, enthalten.

Gleichermaßen werden gute Therapieerfolge erzielt, wenn das Medikament 14 eine Kombination von mindestens zwei, vorzugsweise mindestens drei, voneinander verschiedenen pharmazeutischen Wirkstoffen enthält, wobei die Kombination mindestens zwei Wirkstoffe aus der Gruppe von Dexpanthenol, Polyhexanid, Octenidin, Chlorhexidin, Allantoin, Chondroitin oder Chondroitinsulfat, Hyaluronsäure oder deren Salzen, Oxybutynin, Bromelain und Aescin enthält und/oder wobei die Kombination mindestens ein Wundheilmittel, vorzugsweise Dexpanthenol, zusammen mit mindestens einem weiteren Wirkstoff aus der Gruppe von Polyhexanid, Octenidin, Chlorhexidin, Allantoin, Chondroitin oder Chondroitinsulfat, Hyaluronsäure oder deren Salzen, Oxybutynin, Bromelain und Aescin enthält.

Das Medikament 14 kann in flüssiger oder fließfähiger Form, vorzugsweise in Form einer mindestens einen pharmazeutischen Wirkstoff enthaltenden flüssigen Zusammensetzung, ausgebildet sein.

Gemäß einer weiteren Ausführungsform kann das Medikament 14 in viskoser und/oder gelförmiger Form ausgebildet sein und/oder in Form eines Gels vorliegen.

Weiterhin kann das Medikament 14 bei einer Temperatur von 25 °C eine Brookfield-Viskosität im Bereich von 500 bis 100.000 mPas, insbesondere 750 bis 10.000 mPas, bevorzugt 1.000 bis 7.500 mPas, besonders bevorzugt 1.250 bis 5.000 mPas, ganz besonders bevorzugt 1.300 bis 3.000 mPas, aufweisen.

Des Weiteren kann es vorgesehen sein, dass das Medikament 14 bei einer Temperatur von 25 °C und bei Atmosphärendruck (1.013 hPa) eine Dichte im Bereich von 0,7 bis 1,5 g/cm³, insbesondere 0,8 bis 1,4 g/cm³, bevorzugt 0,85 bis 1,3 g/cm³, besonders bevorzugt 0,9 bis 1,2 g/cm³, ganz besonders bevorzugt 0,95 bis 1,1 g/cm³, aufweist.

Außerdem kann es vorgesehen sein, dass das Medikament 14 zusätzlich mindestens einen Gelbildner aufweist, insbesondere wobei der Gelbildner ausgewählt ist aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen, vorzugsweise Cellulose und Cellulosederivaten, insbesondere modifizierten Cellulosen, bevorzugt Methylcellulose, Carboxymethylcellulose und/oder Hydroxyethylcellulose.

Schließlich kann das Medikament 14 einen physiologisch verträglichen pH-Wert aufweisen. Insbesondere kann das Medikament 14 einen pH-Wert im Bereich von 4 bis 8, bevorzugt 5 bis 7,5, besonders bevorzugt 6 bis 7, aufweisen.

Was die erfindungsgemäß eingesetzte Applikationsvorrichtung 27 anbelangt, so kann deren Volumen in weiteren Bereichen variieren: Insbesondere kann es vorgesehen sein, dass die Applikationsvorrichtung 27 ein Volumen im Bereich 10 bis 300 ml, insbesondere 15 bis 150 ml, vorzugsweise 20 bis 100 ml, aufweist. Weiterhin kann die erfindungsgemäß eingesetzte Applikationsvorrichtung 27 das Medikament 14 in volumenbezogenen Mengen von 10 bis 300 ml, insbesondere 15 bis 150 ml, vorzugsweise 20 bis 100 ml, enthalten. Dabei kann das Volumen und/oder die volumenbezogene Menge in Abhängigkeit von der Größe der zu behandelnden Prostata 3 und/oder der ausrezesierten Prostataloge ausgewählt werden.

Gemäß einer Ausführungsform der vorliegenden Erfindung kann es vorgesehen sein, dass eine einzige Applikationsvorrichtung 27 nur zur Befüllung des Prostataballons 16 oder aber zur Befüllung sowohl des Prostataballons 16 als auch des Blasenballons 12 vorgesehen ist.

Gemäß einer hierzu alternativen Ausführungsform kann es jedoch auch vorgesehen sein, dass zwei unterschiedliche Applikationsvorrichtungen 27 zur Befüllung des Prostataballons 16 einerseits und des Blasenballons 12 andererseits vorgesehen sind. Dabei kann eine erste, zur Befüllung des Prostataballons 16 bestimmte Applikationsvorrichtung mindestens ein wie zuvor definiertes Medikament 14 enthalten. Eine zweite, zur Befüllung des Blasenballons 12 bestimmte Applikationsvorrichtung kann dagegen mindestens ein wie zuvor definiertes Medikament 14, gleich oder verschieden zu dem Medikament 14 in der ersten Applikationsvorrichtung, wobei das Medikament 14 in der zweiten Applikationsvorrichtung insbesondere Hyaluronsäure oder deren Salze und/oder Chondroitinsulfat umfasst, oder aber nur Wasser oder eine wässrige Kochsalzlösung (z. B. sogenannte Ringerlösung) enthalten.

Insbesondere kann es erfindungsgemäß vorgesehen sein, dass unterschiedliche Zusammensetzungen für die Befüllung des Prostataballons 16 einerseits und für die Befüllung des Blasenballons 12 andererseits vorgesehen sind; dabei ist es bevorzugt, wenn zur Befüllung des Prostataballons 16 ein wie zuvor definiertes Medikament 14 eingesetzt wird. Gleichermaßen ist es gemäß einer alternativen Ausführungsform aber auch möglich, dass eine gleiche bzw. identische Zusammensetzung für die Befüllung des Prostataballons 16 einerseits und für die Befüllung des Blasenballons 12 andererseits vorgesehen ist.

Um eine sichere Anwendung zu gewährleisten, ist es erfindungsgemäß bevorzugt, wenn im Rahmen des erfindungsgemäßen Kit 28 der Katheter 1 einerseits und die Applikationsvorrichtung 27 andererseits mit bzw. in einer gemeinsamen Umverpackung (nicht dargestellt) vorliegen, insbesondere zusammen mit technischen Anwendungshinweisen (z. B. in Papierform oder in elektronischer Form).

Weitere Ausgestaltungen, Abwandlungen und Variationen der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung aber nicht beschränken sollen.

### Ausführungsbeispiele:

### 1. In-vitro-Studie zur Untersuchung des Einflusses der Wirksubstanzen auf die Wundheilung

Der Einfluss der erfindungsgemäß eingesetzten Wirksubstanzen auf die Wundheilung in vitro wurde im Rahmen einer an die Methode von *Liang et al.* (2007) angelehnten Studie untersucht. Dazu wurden Fibroblasten in einem geschlossenen (lückenlosen) Monolayer (Zellrasen) kultiviert. Anschließend wurde eine definierte "Wunde" erzeugt, indem eine Lücke in den Zellrasen gekratzt wurde. Durch die Beobachtung und Messung des Zuwachsens der Wunde unter der Behandlung konnten verschiedene Testrezepturen in Bezug auf die Förderung des Heilungsprozesses miteinander verglichen werden.

### a) Testrezepturen

Allen Testrezepturen wurde als Exzipient (Träger) eine Ringerlösung zugrundegelegt. Die übrigen eingesetzten Wirkstoffe sind der nachfolgenden Tabelle 1 zu entnehmen.

**Tabelle 1: Testrezepturen**

| **Rezeptur (Bezeichnung)** | **Wirkstoffe** |
|---|---|
| A | 0,1 Gew.-% Allantoin |
| | 0,01 Gew.-% Polyhexanid |
| B | 2,5 Gew.-% Dexpanthenol |
| | 0,1 Gew.-% Polyhexanid |
| C | 2,5 Gew.-% Dexpanthenol |
| | 0,01 Gew.-% Polyhexanid |
| D | 2,5 Gew.-% Dexpanthenol |
| | 0,01 Gew.-% Polyhexanid |
| | 0,1 Gew.-% Allantoin |
| E | 0,1 Gew.-% Allantoin |
| F | 2,5 Gew.-% Dexpanthenol |
| G | Negativkontrolle (reine Ringerlösung) |

### b) Vorgehensweise

Im Rahmen der vorliegenden in-vitro-Studie wurde das Zuwachsen bzw. Abheilen der "Wunde" im Fibroblasten-Monolayer unter dem Einfluss der Testsubstanzen beobachtet und das Zuwachsen der "Wunde" gemessen. Aus diesen Messungen wurde die Wirksamkeit der Testsubstanzen im Vergleich zu Positivkontrollen sowie im Vergleich zu unbehandelten Kontrollen bestimmt.

Zur Analyse des Einflusses der Testrezepturen auf die Geschwindigkeit der Wundheilung wurden Fibroblasten in Petrischalen ausgesät und bis zur Ausbildung eines geschlossenen Monolayers kultiviert. In diesen Monolayer wurde mit einer Pipettenspitze (100 bis 300 µl) eine Wunde gekratzt (sogenannter "Scratch-Test" oder "Kratztest"). In unmittelbarem Anschluss wurden die Zellen mit den Testformulierungen supplementiert bzw. in Kontakt gebracht und einwirken gelassen.

Zum Zeitpunkt der "Verwundung" (Zeitpunkt = 0 Stunden) und nach 6 bis 8 Stunden wurden Fotografien derselben markierten verwundeten Stellen angefertigt und computergestützt ausgewertet. Unmittelbar nach der "Verletzung" bilden die scharfen "Wundränder" eine klare Begrenzung zwischen Wundfläche und Zellrasen. Als bester Zeitpunkt für die Messung wurde der relativ frühe Zeitraum zwischen 6 und 8 Stunden herangezogen, da hier die Wunde noch klar erkennbar war und auch die einwachsenden Zellen noch klar begrenzt am Rand der Wunde lokalisiert waren. Die geschlossene Front der neuen Fibroblasten wurde als Grenze der Wundfläche definiert. Zu späteren Zeitpunkten löste sich die klare Begrenzung auf und mehr und mehr Zellen siedelten sich in der Mitte der freien Fläche an.

Im Anschluss wurden die zellfreien Flächen, d. h. die Fläche der jeweiligen Wunde zum Zeitpunkt der "Verwundung", mit Hilfe eines Bildverarbeitungsprogramms vermessen und mit der zellfreien Fläche des zweiten Zeitwertes verglichen. Auf Basis der Differenz der Flächen wurde die Wundheilungsförderung durch die Testrezepturen A bis F bzw. durch die Negativkontrolle G mit einem Schulnotensystem (1 = "sehr gut", d.h. exzellente "Wundheilung", bis 6 = "ungenügend", d. h. keine "Wundheilung") bewertet. Diesbezügliche Details sind dem nachfolgenden Abschnitt zu entnehmen.

### c) Auswertung und Ergebnisse

Wie zuvor erwähnt, wurden die zellfreien Flächen des Fibroblasten-Monolayers zum Zeitpunkt der "Verwundung" sowie 8 Stunden nach der "Verwundung" gemessen und im Anschluss miteinander verglichen. Je stärker die jeweilige Testrezeptur die Wundheilung fördert, desto kleiner war 8 Stunden nach der "Verwundung" die zellfreie Fläche ausgebildet, d. h. desto stärker war die künstlich erzeugte Wunde bereits wieder "verheilt". Die Wundheilungsförderung durch die Testrezepturen wurde mit dem Schulnotensystem bewertet. Die diesbezüglich erhaltenen Ergebnisse sind der nachfolgenden Tabelle 2 zu entnehmen.

**Tabelle 2: Wundheilung bei Verwendung der Testrezepturen**

| **Rezeptur** | **Wirkstoffe** | **Benotung** |
|---|---|---|
| A | 0,1 Gew.-% Allantoin | 3 |
| | 0,01 Gew.-% Polyhexanid | |
| B | 2,5 Gew.-% Dexpanthenol | 1 - 2 |
| | 0,1 Gew.-% Polyhexanid | |
| C | 2,5 Gew.-% Dexpanthenol | 1 - 2 |
| | 0,01 Gew.-% Polyhexanid | |
| D | 2,5 Gew.-% Dexpanthenol | 1 |
| | 0,01 Gew.-% Polyhexanid | |
| | 0,1 Gew.-% Allantoin | |
| E | 0,1 Gew.-% Allantoin | 3 - 4 |
| F | 2,5 Gew.-% Dexpanthenol | 2 |
| G | Negativkontrolle (reine Ringerlösung) | 5 - 6 |

Aus der obigen Tabelle geht hervor, dass mit Rezeptur D auf Basis von Dexpanthenol, Polyhexanid und Allantoin die Vermehrung der Zellen bzw. der Heilungsprozess am stärksten gefördert werden konnte. Eine nur geringfügig schwächere Wundheilungsförderung wurde mit den Rezepturen B bzw. C auf Basis von Dexpanthenol und 0,1 Gew.-% Polyhexanid bzw. Dexpanthenol und 0,01 Gew.-% Polyhexanid erzielt. Weniger stark, aber dennoch zufriedenstellend, konnte die Wundheilung mit einer Kombination von Allantoin und Polyhexanid (Rezeptur A) gefördert werden. Darüber hinaus lässt sich aus den obigen Ergebnissen entnehmen, dass überraschenderweise bereits durch den alleinigen Einsatz von Dexpanthenol (Rezeptur F) die Wundheilung verhältnismäßig stark gefördert werden kann. Der alleinige Einsatz von Allantoin (Rezeptur E) hingegen führte nur zu einer allenfalls befriedigenden bis ausreichenden Förderung der Wundheilung. Wurde nur Ringerlösung ohne jegliche Wirkstoffe eingesetzt, konnte keine nennenswerte Förderung der Wundheilung verzeichnet werden.

### d) Schlussfolgerungen der in-vitro-Studie

Formulierungen mit Dexpanthenol, insbesondere in Kombination mit Polyhexanid und/oder Allantoin, führen zu einer effizienten Förderung der Wundheilung bzw. zu einer besonders schnellen Regeneration der Verletzung. Eine Formulierung mit Allantoin allein und eine Kombination von Allantoin mit Polyhexanid fördern zwar ebenfalls die Wundheilung, allerdings nicht in dem Maße wie dexpanthenolhaltige Formulierungen. Die beste Wundheilungsförderung konnte mit einer Kombination auf Basis von Dexpanthenol, Allantoin und Polyhexanid erzielt werden.

### 2. Prospektiv randomisierte Studie zur Untersuchung der Wirksamkeit des erfindungsgemäßen Katheters und des erfindungsgemäßen Kathetersystems

In einer prospektiv randomisierten Studie wurde zudem untersucht, ob es unter Einsatz der obigen Testrezepturen nach einer Behandlung mit einem erfindungsgemäßen transurethralen zweifach medikamentös beschickten Doppelballon-Katheter (d. h. Prostata- und Blasenballon) zu einer besseren Abheilung des Resektionsbetts nach transurethraler Resektion oder Vaporisation der Prostata kommt.

Dazu wurden 6 Gruppen mit jeweils 10 männlichen Patienten im Alter zwischen 69 und 87 Jahren gebildet, bei denen die Indikation zur transurethralen Resektion oder Vaporisation der Prostata besteht. Bei jeder der Gruppen wurde der Ballon-Katheter jeweils mit einer der Testrezepturen A, C, D, E oder F beschickt, wobei hierzu jeweils ein anwendungsfertiges erfindungsgemäßes Kit aus einer mit der jeweiligen Testrezeptur gefüllten Applikationsvorrichtung in Form einer Spritze und einem Katheter mit permeablem Prostata- und Blasenballon (jeweils laserperforiert mittels Ultrakurzpulslaser, jeweils 4 bis 6 Poren mit Durchmessern von 5 bis 30 µm) zur Anwendung kam. Bei der sechsten Gruppe wurde zu Vergleichszwecken der Katheter lediglich mit wirkstofffreier Ringerlösung beschickt; zusätzlich erhielten fünf der Probanden der sechsten Gruppe über den Zeitraum der Katheterisierung systemisch 3-mal täglich 600 mg Ibuprofen, während die übrigen fünf Probanden dieser Gruppe systemisch 3-mal täglich 30 Tropfen Novaminsulfon (Metamizol) erhielten.

Zu unterschiedlichen Zeitpunkten (T1 bis T6) wurde evaluiert, ob es zwischen den Gruppen zu einer unterschiedlichen Reduzierung in Bezug auf die in der Literatur bekannten postoperativen Störungen, wie Dysurie, irritative Symptomatik, vorübergehende Inkontinenz, Blasenspasmen und Harnverhalt kommt. Darüber hinaus wurde mit Hilfe des *International Prostate Symptom Score* (IPSS) evaluiert, ob es zwischen den Gruppen einen Unterschied gibt. Die unterschiedlichen Wundheilungen der Resektionsflächen wurden makroskopisch per Zystoskopie erfasst.

### a) Studienablauf

Nachfolgend ist der gesamte Studienablauf im Detail beschrieben. Im Hinblick auf die Beurteilung der Wundheilungsförderung durch die Testrezepturen sind insbesondere der Symptomen-Score sowie die Beurteilung der Schmerzen anhand der visuellen Analogskala von Bedeutung. Die übrigen Analysen wurden zur Überwachung der Vitalfunktionen der Patienten durchgeführt und sind der Vollständigkeit halber an dieser Stelle aufgeführt.

**T1 (präoperative Phase)**

| | |
|---|---|
| Aufnahmestatus: | digital-rektale Untersuchung, Miktionstatus (Tages- und Nachtmiktion), Erektionstatus |
| Labor: | Blutbild, Kreatinin, Harnstoff, Elektrolyte, Transaminasen |
| Urin: | urinchemische und -mikroskopische Untersuchung, Urinkultur, spezifisches Gewicht |
| Uroflowmetrie: | maximaler und mittlerer Flow, Volumen, Restharn |
| Sonographie: | Abdomen, transrektale Prostatasonographie (TRUS) |
| Symptomen-Score: | IPSS |
| Schmerzen: | Visuelle Analogskala |

**T2 (2 Tage nach der transurethralen Prostataresektion bzw. Vaporisation)**

| | |
|---|---|
| Urin: | urinchemische und urinmikroskopische Untersuchung, Urinkultur, spezifisches Gewicht |
| Schmerzen: | Visuelle Analogskala |

**T3 (1 Tag nach Entfernung des Katheters)**

| | |
|---|---|
| Uroflowmetrie: | maximaler und mittlerer Flow, Volumen, Restharn |
| Schmerzen: | Visuelle Analogskala |

**T4 (2 Wochen nach Entfernung des Katheters)**

| | |
|---|---|
| Untersuchungsstatus: | Miktionstatus (Tages- und Nachtmiktion), Erektionstatus |
| Labor: | Blutbild, Kreatinin, Harnstoff, Elektrolyte, Transaminasen |
| Urin: | urinchemische und -makroskopische Untersuchung, Urinkultur, spezifisches Gewicht |
| Uroflowmetrie: | maximaler und mittlerer Flow, Volumen, Restharn |
| Sonographie: | Abdomen |
| Symptomen-Score: | IPSS |
| Schmerzen: | Visuelle Analogskala |

**T5 (3 Monate nach Entfernung des Katheters)**

| | |
|---|---|
| Untersuchungsstatus: | digital-rektale Untersuchung, Miktionstatus (Tages- und Nachtmiktion), Erektionstatus |
| Labor: | Blutbild, Kreatinin, Harnstoff, Elektrolyte, Transaminasen |
| Urin: | urinchemische und -makroskopische Untersuchung, Urinkultur, spezifisches Gewicht |
| Uroflowmetrie: | maximaler und mittlerer Flow, Volumen, Restharn |
| Sonographie: | Abdomen, transrektale Prostatasonographie (TRUS) |
| Symptomen-Score: | IPSS |
| Schmerzen: | Visuelle Analogskala |
| Zystoskopie: | makroskopische Dokumentation der Wundabheilung |

**T6 (6 Monate nach Entfernung des Katheters)**

| | |
|---|---|
| Symptomen-Score: | IPSS |

### b) Ergebnisse (Symptomen-Score)

Zur Ermittlung des Symptomen-Score gemäß dem *International Prostate Symptom Score* (IPSS) füllten die Probanden zu den oben genannten Zeitpunkten T1, T4, T5 und T6 den diesbezüglichen standardisierten und validierten Fragebogen aus. Die sich aus dem ausgefüllten Fragebogen ergebende Punktzahl gemäß WHO führte dabei zu der nachfolgend geschilderten Kategorisierung in eine milde, mittlere oder schwere Symptomatik in Bezug auf Beschwerden des unteren Harntrakts:

| | |
|---|---|
| 0 - 7 Punkte: | Die Beschwerden des unteren Harntrakts sind nach offizieller Einteilung der milden Symptomatik zugeordnet. Es sollte eine Rücksprache mit einem Arzt erfolgen. Darüber hinaus sollte der Test in vier Wochen wiederholt werden. |
| 8 - 19 Punkte: | Die Beschwerden des unteren Harntraktes stellen eine starke Beeinträchtigung dar und sind nach offizieller Einteilung der mittleren Symptomatik zugeordnet. Es sollte eine baldige Rücksprache mit einem Arzt erfolgen. |
| 20 - 35 Punkte: | Die Beschwerden des unteren Harntraktes stellen eine sehr starke Beeinträchtigung und sind nach offizieller Einteilung der schweren Symptomatik zugeordnet. Es sollte eine umgehende Rücksprache mit einem Arzt erfolgen. |

Die erhaltenen Ergebnisse sind der nachfolgenden Tabelle 3 zu entnehmen:

**Tabelle 3: Ergebnisse der IPSS-Auswertung**

| | **T1** | **T4** | **T5** | **T6** |
|---|---|---|---|---|
| **Gruppe 1 (Formulierung A)** | | | | |
| Patient 1 | 32 | 20 | 18 | 5 |
| Patient 2 | 27 | 23 | 16 | 6 |
| Patient 3 | 33 | 22 | 19 | 2 |
| Patient 4 | 35 | 25 | 15 | 9 |
| Patient 5 | 27 | 19 | 13 | 11 |
| Patient 6 | 24 | 23 | 14 | 7 |
| Patient 7 | 29 | 19 | 16 | 13 |
| Patient 8 | 31 | 17 | 17 | 10 |
| Patient 9 | 33 | 23 | 14 | 12 |
| Patient 10 | 35 | 22 | 13 | 7 |
| **Mittelwert** | **30,6** | **21,3** | **15,5** | **8,2** |

| **Gruppe 2 (Formulierung C)** | | | | |
|---|---|---|---|---|
| Patient 1 | 35 | 21 | 11 | 2 |
| Patient 2 | 33 | 18 | 7 | 6 |
| Patient 3 | 32 | 19 | 5 | 3 |
| Patient 4 | 27 | 22 | 8 | 7 |
| Patient 5 | 25 | 24 | 5 | 3 |
| Patient 6 | 29 | 16 | 10 | 1 |
| Patient 7 | 26 | 20 | 9 | 2 |
| Patient 8 | 31 | 21 | 6 | 0 |
| Patient 9 | 27 | 23 | 7 | 5 |
| Patient 10 | 30 | 19 | 8 | 4 |
| **Mittelwert** | **29,5** | **20,3** | **7,6** | **3,3** |

| **Gruppe 3 (Formulierung D)** | | | | |
|---|---|---|---|---|
| Patient 1 | 31 | 17 | 4 | 1 |
| Patient 2 | 35 | 20 | 6 | 3 |
| Patient 3 | 26 | 13 | 4 | 4 |
| Patient 4 | 27 | 14 | 2 | 0 |
| Patient 5 | 25 | 12 | 1 | 0 |
| Patient 6 | 32 | 19 | 5 | 2 |
| Patient 7 | 35 | 16 | 2 | 0 |
| Patient 8 | 30 | 25 | 4 | 3 |
| Patient 9 | 27 | 20 | 8 | 6 |
| Patient 10 | 24 | 17 | 3 | 0 |
| **Mittelwert** | **29,2** | **17,3** | **3,9** | **1,9** |

| **Gruppe 4 (Formulierung E)** | | | | |
|---|---|---|---|---|
| Patient 1 | 33 | 23 | 12 | 9 |
| Patient 2 | 27 | 24 | 15 | 12 |
| Patient 3 | 26 | 19 | 15 | 15 |
| Patient 4 | 29 | 25 | 17 | 8 |
| Patient 5 | 28 | 20 | 10 | 5 |
| Patient 6 | 35 | 27 | 21 | 11 |
| Patient 7 | 32 | 21 | 11 | 7 |
| Patient 8 | 26 | 18 | 20 | 13 |
| Patient 9 | 31 | 20 | 14 | 9 |
| Patient 10 | 29 | 27 | 16 | 10 |
| **Mittelwert** | **29,6** | **22,4** | **15,1** | **9,9** |

| **Gruppe 5 (Formulierung F)** | | | | |
|---|---|---|---|---|
| Patient 1 | 32 | 21 | 8 | 5 |
| Patient 2 | 29 | 25 | 6 | 6 |
| Patient 3 | 25 | 17 | 7 | 3 |
| Patient 4 | 27 | 22 | 11 | 7 |
| Patient 5 | 35 | 25 | 5 | 3 |
| Patient 6 | 24 | 21 | 4 | 1 |
| Patient 7 | 32 | 20 | 12 | 9 |
| Patient 8 | 31 | 19 | 4 | 2 |
| Patient 9 | 26 | 17 | 7 | 5 |
| Patient 10 | 30 | 23 | 5 | 4 |
| **Mittelwert** | **29,1** | **21** | **6,9** | **4,5** |

| **Gruppe 6 (Formulierung G + Ibuprofen bzw. Novaminsulfon)** | | | | |
|---|---|---|---|---|
| Patient 1 (Ibuprofen) | 26 | 25 | 21 | 18 |
| Patient 2 (Ibuprofen) | 31 | 29 | 23 | 19 |
| Patient 3 (Ibuprofen) | 27 | 27 | 19 | 22 |
| Patient 4 (Ibuprofen) | 24 | 22 | 21 | 19 |
| Patient 5 (Ibuprofen) | 35 | 34 | 29 | 15 |
| Patient 6 (Novamin.) | 32 | 29 | 27 | 16 |
| Patient 7 (Novamin.) | 29 | 29 | 22 | 12 |
| Patient 8 (Novamin.) | 33 | 31 | 25 | 20 |
| Patient 9 (Novamin.) | 31 | 26 | 25 | 21 |
| Patient 10 (Novamin.) | 27 | 22 | 20 | 13 |
| **Mittelwert** | **29,5** | **27,4** | **23,2** | **17,5** |

Aus den vorstehenden Ergebnissen geht hervor, dass sich mit Testrezeptur D, welche eine Kombination auf Basis von Dexpanthenol, Polyhexanid und Allantoin enthält, die Wundheilung der Prostata nach transurethralen Resektionen bzw. Vaporisationen am wirkungsvollsten fördern lässt. Bei der Patientengruppe, welche die Testrezeptur D erhielt, war bereits 2 Wochen nach der postoperativen Entnahme des Katheters die Miktion im Vergleich zum Zustand vor der Operation verbessert. Drei Monate nach der Operation hatten sich die Beschwerden des unteren Harntrakts bei einem Großteil der Patienten vollständig zurückgebildet, d. h. die Beschwerden des unteren Harntrakts konnten einer milden Symptomatik zugeordnet werden. Bis zum Zeitpunkt T6 entsprechend sechs Monaten nach der Operation wurde noch ein weiterer Rückgang der Beschwerden verzeichnet.

Ähnlich gute Ergebnisse wurden bei der Patientengruppe erzielt, welche Dexpanthenol in Kombination mit Polyhexanid erhalten hatte. Auch bei diesen Patienten waren die Beschwerden innerhalb eines Zeitraums von drei Monaten nach der Operation weitgehend zurückgegangen. Bis zum Zeitpunkt von sechs Monaten nach der Operation gingen die Beschwerden noch weiter zurück.

Wie sich bereits überraschenderweise im Rahmen der in-vitro-Studie an Fibroblasten gezeigt hatte, wurden auch bei alleinigem Einsatz von Dexpanthenol gute Ergebnisse in Bezug auf die Wundheilungsförderung nach Prostataresektionen bzw. -vaporisationen erhalten.

### c) Schlussfolgerungen und weitere Ergebnisse

Mit dem erfindungsgemäßen Katheter und dem erfindungsgemäßen Kathetersystem (Kit) lässt sich eine wirksame lokale kausale Therapie in Bezug auf die Behandlung von Funktionsstörungen bzw. Erkrankungen der Prostata und der Blase, insbesondere die medikamentöse und physikalische Behandlung der Prostata und der Blase in Verbindung mit bzw. nach einer transurethralen Resektion der Prostata, realisieren.

Es kommt zu einer statistisch signifikanten Reduzierung des IPSS in den Therapie-Gruppen (d. h. obige Gruppen 1 bis 5) im Vergleich zur Kontroll-Gruppe (d. h. obige Gruppe 6). Die mit Hilfe der visuellen Analogskala ermittelten Schmerzen reduzieren sich in den Therapie-Gruppen im Vergleich zur Kontroll-Gruppe zudem deutlich. Dies bedeutet, dass sich die Lebensqualität als entscheidender Parameter für die Ergebnisqualitätssicherung bei den erfindungsgemäß behandelten Patienten deutlich verbessert. Durch die Reduzierung der lang anhaltenden Miktionsbeschwerden verliert das operative Therapieverfahren deutlich an negativen Langzeitfolgen. Es ist daher bezüglich alternativer Therapieverfahren deutlich konkurrenzfähiger.

Bei der makroskopischen Betrachtung des Resektionsbettes nach erfolgter transurethraler Resektion der Prostata (TUR-P) kommt es zudem zu einer deutlichen Reduzierung der Fibrinbildung bzw. Fibrinreduzierung in der Therapie-Gruppe, verbunden mit einer deutlich besseren Wundabheilung. In der Urin-Mikroskopie weisen die Therapie-Gruppen eine deutlich geringere Leukozyturie und Mikrohämaturie auf als die Kontroll-Gruppe. In den Therapie-Gruppen kommt es zu keiner Zunahme von Harnwegsinfekten. Durch die bessere Wundabheilung in den Therapie-Gruppen ist die Bakteriurie nämlich deutlich reduziert.

In der Uroflometrie weisen die Therapie-Gruppen einen deutlich verbesserten maximalen und mittleren Flow sowie eine signifikante Zunahme des Blasenvolumens im Vergleich zur Kontroll-Gruppe auf. Ebenfalls kommt es zu einer Reduzierung der Restharnmenge bei den erfindungsgemäß behandelten Patienten.

Die Miktionsfrequenz reduziert sich diurn und nokturn durch die erfindungsgemäße lokale Behandlung, bedingt durch die bessere Abheilung des Wundbetts.

Die Laborwerte dagegen bewegen sich sowohl in den Therapie-Gruppen als auch in der Kontroll-Gruppe nicht im pathologischen Bereich. In allen Gruppen kommt es in der Sonographie zu keinen Harnabfluss-Störungen. Der Erektionsstatus verschlechtert sich in der Kontroll-Gruppe durch die Miktionsstörungen in Form von Dysurie und Pollakisurie sowie eine ausgeprägten Drangsymptomatik. In den Therapie-Gruppen dagegen tritt durch eine bessere Wundabheilung des Resektionsbetts keine Verschlechterung des Erektionsstatus ein.

### 3. Untersuchungen zum Permeations-/Diffusionsverhalten erfindungsgemäßer und nichterfindungsgemäßer Katheter

Weiterhin wurden weiterführende Untersuchungen bzw. Studien zum Permeabilitäts- bzw. Diffusionsverhalten von verschiedenen, als Wandungen des Prostataballons verwendbaren Silikonmembranen durchgeführt.

Hierzu wurden zum einen (a) eine für diesen Zweck handelsübliche, kontinuierliche, homogene und somit nichtperforierte Silikonmembran und zum anderen einerseits (b) eine mittels Laserbehandlung mittels Ultrakurzpulslaser mikroperforierte Silikonmembran, welche Poren mit Größen im Bereich von 5 bis 30 µm aufweist, und andererseits (c) eine im Rahmen ihrer Herstellung *in situ* mikroperforierte Silikonmembran gleichermaßen mit Porengrößen im Bereich von 5 bis 30 µm untersucht.

Zunächst wurde die Permeabilität der jeweiligen Silikonmembranen (a) bzw. (b) bzw. (c) jeweils für eine erste Triclosanlösung, welche Triclosan in Mengen von 0,3 Gew.-%., bezogen auf die Lösung, enthielt, und für eine zweite Triclosan-Lösung, welche Triclosan in Mengen von 1,0 Gew.-%., bezogen auf die Lösung, enthielt, ermittelt. Für den zugrundeliegenden Versuchszeitraum von zehn Tagen wurde bei (a) der kontinuierlichen, nichtperforierten Silikonmembran bei der niedrig konzentrierten Triclosan-Lösung keine nennenswerte Freisetzung von Triclosan festgestellt, wobei die diesbezüglichen Mengen an über die Silikonschicht freigesetztem Triclosan deutlich unterhalb von 1,5 µg/ml lagen. Demgegenüber konnte in Bezug auf (b) und (c) die mikroperforierten Silikonmembranen jeweils eine signifikant höhere Freisetzung an Triclosan beobachtet werden, welche jeweils mehr als das 10.000-Fache der Menge an Triclosan, welches bei (a) der kontinuierlichen Silikonmembran freigesetzt wurde, betrug. Entsprechende Ergebnisse wurden in vergleichbarer Größenordnung auch für die höher konzentrierte Triclosan-Lösung ermittelt.

In diesem Zusammenhang wurde auch eine weitere Versuchsreihe mit Dexpanthenol als Wirkstoff durchgeführt, wobei diesbezüglich eine Dexpanthenollösung eingesetzt wurde, welche Dexpanthenol in Mengen von 2,5 Gew.-%, bezogen auf die Lösung, enthielt. Auch in Bezug auf Dexpanthenol wurden hinsichtlich (a) der kontinuierlichen Silikonmembran keine signifikanten Mengen an Wirkstoff freigesetzt, während bei den mikroperforierten Silikonmembranen (b) und (c) jeweils eine signifikant höhere Freisetzung an Dexpanthenol, welche jeweils mehr als das 5.000fache der nichtperforierten Silikonmembran freigesetzten Menge betrug, festgestellt wurde.

Im Ergebnis verhält es sich somit in Bezug auf (a) die nichtperforierte bzw. kontinuierliche Silikonmembran derart, dass dort eine Freisetzung von Wirksubstanzen in therapeutisch wirksamen Mengen gerade nicht möglich ist. Erst im Rahmen der durchgeführten Modifizierung der Silikonmembran auf Basis einer Mikroperforation (Silikonmembranen (b) und (c)) ist es überhaupt möglich, pharmakologisch relevante Mengen der jeweiligen Wirkstoffe freizusetzen.

Darüber hinaus wurde ein weiterer Freisetzungsversuch auf Basis der Verwendung einer Methylenblau-Lösung durchgeführt. Während in Bezug auf (a) die nichtperforierte bzw. kontinuierliche Silikonmembran keine signifikante Freisetzung bzw. keine signifikante Blaufärbung des externen bzw. äußeren Mediums beobachtet werden konnte, lag für (b) und (c) die mikroperforierten Silikonmembranen bereits nach relativ kurzen Zeiträumen jeweils eine Äquilibrierung vor, d.h. eine gleiche Farbgebung der zugrundeliegenden Flüssigkeiten auf beiden Seiten der Silikonmembranen, was auf signifikant verbesserte bzw. vergrößerte Permeationseigenschaften bei den mikroperforierten Silikonmembranen schließen lässt.

Die seitens des Anmelders durchgeführten weiterführenden Untersuchungen belegen somit, dass eine kontinuierliche Membran trotz einer gewissen Eigendurchlässigkeit nicht dazu geeignet ist, therapeutisch wirksame Substanzen in pharmakologisch relevanten Mengen freizusetzen, was erst durch eine weiterführende Modifizierung in Form einer Mikroperforation ermöglicht wird. Die Ergebnisse zeigen somit, dass eine kontinuierliche Silikonmembran in Bezug auf freizusetzende Wirkstoffe vielmehr als Barriere- bzw. Trennschicht fungiert, welche keine signifikante Freisetzung ermöglicht.

### 4. Untersuchungen zur mechanischen Stabilität in Bezug auf verschiedenartig erzeugte Mikroperforationen

Weiterhin wurden Untersuchungen bzw. Studien zur mechanischen Stabilität in Bezug auf verschiedenartig erzeugte Mikroperforationen durchgeführt.

Hierzu wurden Ballonkatheter auf Basis von Polydimethylsiloxan-Membranen hergestellt, bei welchen die Polydimethylsiloxan-Membranen jeweils auf verschiedene Weise mit Mikroperforationen jeweils mit Porengrößen im Bereich von 5 bis 30 µm versehen waren (Wandungsdicke: jeweils ca. 150 µm, Volumen im expandierten Zustand: jeweils ca. 120 ml):
Bei einer ersten Serie von zehn porösen Ballonkathetern wurde die Mikroporen durch physikomechanische Behandlung bzw. Perforation erzeugt, während bei einer zweiten Serie von zehn Ballonkathetern die Mikroperforation mittels Laserperforation mit Ultrakurzpulslaser erfolgte. Bei einer dritten Serie von weiteren zehn Ballonkathetern waren die Mikroporen herstellungsbedingt *in situ* durch chemische Modifizierung bei der Polymerisation (Polymerisation in Gegenwart von Porenbildnern auf Polyethylenglykolbasis) erzeugt worden. Alle Ballone (d. h. die jeweils zehn Ballone der drei verschiedenen Testserien) wurden mit ihrem jeweiligen Maximalvolumen mit Wasser befüllt und unter dieser Füllbelastung 24 Stunden lang in der Art einer Rüttelbewegung bewegt. Bei der ersten Serie von Ballonkathetern waren bereits nach sechs Stunden in drei der zehn getesteten Katheter erste Einrisse in den Wandungen jeweils ausgehend von den Poren festzustellen, wohingegen im Fall der lasererzeugten porösen Wandungen der zweiten Serie diese Defizite erst nach 8,5 Stunden und bei nur zwei Ballonkathetern auftrat. In der dritten Serie dagegen trat ein Defekt bei einem einzigen Ballon erst nach 14,5 Stunden auf.

Die vorstehenden Ergebnisse belegen, dass sich gegenüber Ballonkathetern, bei welchen die Mikroporen mit physikomechanischen Methoden bzw. mechanischer Perforation hergestellt sind, stabilere Katheter mit mechanisch verbesserten Eigenschaften (d. h. einreißfesten Mikroporen) entweder durch Laserperforation oder aber alternativ durch *in-situ*-Generierung der Mikroporen während des Herstellungsprozesses der Wandung realisieren lassen. Mit anderen Worten führen die Laserperforation sowie die im Rahmen der Membranherstellung durchgeführte *in-situ*-Erzeugung der Mikroporen zu mechanisch stabileren, insbesondere einreißfesteren Prostataballonen im Vergleich zu entsprechenden Systemen mit nachträglich mittels mechanischer Methoden erzeugten Poren.

**Bezugszeichenliste:**

| | | | |
|---|---|---|---|
| 1 | Katheter | 16 | Prostataballon |
| 2 | Blase | 17 | Wandung (Prostataballon) |
| 3 | Prostata | 18 | Auslassöffnung (Blasenballon) |
| 4 | Katheterschlauch | 19 | Auslassöffnung (Prostataballon) |
| 5 | distales Ende | 20 | Spitze |
| 5' | Katheteranschluss | 21 | Harnröhre |
| 6 | Mantel | 22 | Stöpsel |
| 7 | proximales Ende | 23 | Ventil (dritter Kanal) |
| 8 | erster Kanal | 24 | Ventil (zweiter Kanal) |
| 9 | Urin | 25 | Poren (Prostataballon) |
| 10 | Ablauföffnung | 26 | Poren (Blasenballon) |
| 11 | dritter Kanal | 27 | Applikationsvorrichtung (Spritze) |
| 12 | Blasenballon | 28 | Kit (Set) |
| 13 | Wandung (Blasenballon) | | |
| 14 | Medikament | a | lichter Abstand |
| 15 | zweiter Kanal | | |

## Patentansprüche

1. Kit (Kit-of-parts oder Set) (28) in Form eines Kathetersystems zur medikamentösen und physikalischen Behandlung der Prostata (3) und gegebenenfalls der Blase (2), vorzugsweise in Verbindung mit oder nach einer transurethralen Resektion und/oder Vaporisation der Prostata (3), in Verbindung mit einer benignen Hyperplasie der Prostata (3) oder in Verbindung mit oder nach einer Bestrahlungstherapie eines Karzinoms der Prostata (3),
wobei das Kit (28) umfasst:
• einerseits einen Katheter (1) in Form eines Verweilkatheters,
wobei der Katheter (1)
- einen Katheterschlauch (4),
- einen am Katheterschlauch (4) befindlichen Katheteranschluss (5') und
- einen befüllbaren und/oder expandierbaren Prostataballon (16),
aufweist,
wobei der Katheterschlauch (4) ein distales Ende (5) und ein proximales Ende (7) aufweist, wobei sich der Katheteranschluss (5') am distalen Ende (5) befindet, wobei ein vom Katheteranschluss (5') bis zu wenigstens einer Ablauföffnung (10) im Bereich des proximalen Endes (7) des Katheterschlauchs (4) reichender erster Kanal (8) vorhanden ist, wobei sich der Prostataballon (16) am proximalen Ende (7) des Katheterschlauchs (4) befindet, wobei ein vom Katheteranschluss (5') bis zu dem Prostataballon (16) im Bereich des proximalen Endes (7) des Katheterschlauchs (4) reichender zweiter Kanal (15) vorhanden ist,
wobei der Prostataballon (16) eine mittels Poren (25) permeabel ausgebildete Wandung (17) aufweist, wobei der Durchmesser der Poren (25) eingestellt ist derart, dass im eingeführten Zustand des Katheters (1) eine gezielte Abgabe eines Medikaments (14) durch die Wandung (17) hindurch erfolgen kann, wobei die Wandung (17) des Prostataballons (16) eine Mehrzahl von Poren (25) mit einem Durchmesser im Bereich von 1 bis 100 µm im expandierten Zustand aufweist und wobei die Poren (25) durch Laserperforation erzeugt sind, und
wobei im Bereich des proximalen Endes (7) des Katheterschlauchs (4) Mittel (12) zur Positionierung des proximalen Endes (7) vorhanden sind, wobei als Mittel (12) zur Positionierung des proximalen Endes (7) ein expandierbarer und/oder befüllbarer Blasenballon (12) vorhanden ist;
und
• andererseits mindestens eine mindestens ein Medikament (14) enthaltende Applikationsvorrichtung (27) zur Verabreichung des Medikaments (14), insbesondere in Form einer vorzugsweise sterilen Spritze, bevorzugt Kolbenspritze, insbesondere zur Befüllung des Katheters (1), insbesondere des Prostataballons (16) und gegebenenfalls des Blasenballons (12), mit dem Medikament (14),
wobei das Medikament (14) mindestens einen pharmazeutischen Wirkstoff enthält, wobei der pharmazeutische Wirkstoff ausgewählt ist aus der Gruppe von Antiphlogistika (Antiinflammatorika), Antiödematika und Wundheilmitteln sowie deren Mischungen und Kombinationen.

2. Kit nach Anspruch 1, **dadurch gekennzeichnet,**
**dass** der pharmazeutische Wirkstoff ausgewählt ist aus der Gruppe von
(i) Antiphlogistika, ausgewählt aus Serrapeptase, Allantoin, Coxibe, Acetylsalicylsäure, Ibuprofen, Kortikosteroiden, insbesondere Hydrocortison, Prednisolon, Methylprednisolon, Budesonid und/oder Dexamethason, und Diclofenac sowie deren Mischungen und Kombinationen, vorzugsweise Allantoin;
(ii) Antiödematika, ausgewählt aus Bromelain und Aescin sowie deren Mischungen und Kombinationen;
(iii) Spasmolytika, insbesondere ausgewählt aus Butylscopolamin (Buscopan), Hyoscyamin, Scopolamin und Ipratropium sowie deren Mischungen und Kombinationen;
(iv) Anticholinergika, insbesondere ausgewählt aus Trospiumchlorid, Tolterodin, Darifenacin, Solifenacin, Oxybutynin und Propiverin sowie deren Mischungen und Kombinationen, vorzugsweise Trospiumchlorid;
(v) Wundheilmitteln, ausgewählt aus Dexpanthenol und dessen Estern, Chondroitin und dessen Salzen, insbesondere Chondroitinsulfat, Zwiebelextrakt, Beinwellextrakt, Hyaluronsäure und deren Salzen, Bisabolol, Ringelblumenextrakt, Kamilleextrakt und Hamamelisextrakt sowie deren Mischungen und Kombinationen, vorzugsweise Dexpanthenol, Chondroitin und dessen Salzen und Hyaluronsäure und deren Salzen;
(vi) Antiseptika, insbesondere ausgewählt aus Chlorhexidin, Octenidin und Polyhexanid sowie deren Mischungen und Kombinationen, vorzugsweise Polyhexanid;
(vii) Lokalanästhetika, insbesondere Lidocain,
sowie deren Mischungen und Kombinationen und/oder
**dass** das Medikament (14) einen Gehalt an Wirkstoff(en) im Bereich von 0,00001 bis 80 Gew.-%, insbesondere 0,0001 bis 50 Gew.-%, vorzugsweise 0,001 bis 25 Gew.-%, bevorzugt 0,005 bis 20 Gew.-%, besonders bevorzugt 0,01 bis 10 Gew.-%, bezogen auf das Medikament (14), aufweist und/oder
**dass** das Medikament (14) neben dem Wirkstoff einen pharmazeutisch kompatiblen Exzipienten, insbesondere Wasser oder eine vorzugsweise isotonische wässrige Kochsalzlösung, insbesondere Ringerlösung, enthält, insbesondere in Mengen im Bereich von 20 bis 99,99999 Gew.-%, insbesondere 50 bis 99,9999 Gew.-%, vorzugsweise 75 bis 99,999 Gew.-%, bevorzugt 80 bis 99,995 Gew.-%, besonders bevorzugt 90 bis 99,99 Gew.-%, bezogen auf das Medikament (14), aufweist.

3. Kit nach Anspruch 1 oder 2, **dadurch gekennzeichnet,**
**dass** das Medikament (14) eine Kombination von mindestens zwei, vorzugsweise mindestens drei, voneinander verschiedenen pharmazeutischen Wirkstoffen enthält, wobei die Kombination mindestens ein Wundheilmittel zusammen mit mindestens einem Antiseptikum und/oder mindestens einem Antiphlogistikum (Antiinflammatorikum) umfasst, vorzugsweise Dexpanthenol zusammen mit Polyhexanid und/oder Allantoin, besonders bevorzugt Dexpanthenol zusammen mit Polyhexanid und gegebenenfalls Allantoin;
insbesondere wobei das Medikament (14), bezogen auf das Medikament (14), Wundheilmittel, insbesondere Dexpanthenol, in Mengen von 0,001 bis 50 Gew.-%, insbesondere 0,01 bis 30 Gew.-%, vorzugsweise 0,1 bis 20 Gew.-%, bevorzugt 0,5 bis 10 Gew.-%, besonders bevorzugt 1 bis 6 Gew.-%, enthält,
insbesondere wobei das Medikament (14), bezogen auf das Medikament (14), Antiseptikum, insbesondere Polyhexanid, in Mengen von 0,0001 bis 20 Gew.-%, insbesondere 0,001 bis 10 Gew.-%, vorzugsweise 0,01 bis 5 Gew.-%, bevorzugt 0,05 bis 2 Gew.-%, besonders bevorzugt 0,05 bis 1 Gew.-%, enthält,
insbesondere wobei das Medikament (14), bezogen auf das Medikament (14), Antiphlogistikum (Antiinflammatorikum), insbesondere Allantoin, in Mengen von 0,0001 bis 20 Gew.-%, insbesondere 0,001 bis 10 Gew.-%, vorzugsweise 0,01 bis 5 Gew.-%, bevorzugt 0,05 bis 2 Gew.-%, besonders bevorzugt 0,05 bis 1 Gew.-%, enthält.

4. Kit nach einem der vorangehenden Ansprüche, **dadurch gekennzeichnet, dass** das Medikament (14) eine Kombination von mindestens zwei, vorzugsweise mindestens drei, voneinander verschiedenen pharmazeutischen Wirkstoffen enthält, wobei die Kombination mindestens zwei Wirkstoffe aus der Gruppe von Dexpanthenol, Polyhexanid, Octenidin, Chlorhexidin, Allantoin, Chondroitin oder Chondroitinsulfat, Hyaluronsäure oder deren Salzen, Oxybutynin, Bromelain und Aescin enthält und/oder wobei die Kombination mindestens ein Wundheilmittel, vorzugsweise Dexpanthenol, zusammen mit mindestens einem weiteren Wirkstoff aus der Gruppe von Polyhexanid, Octenidin, Chlorhexidin, Allantoin, Chondroitin oder Chondroitinsulfat, Hyaluronsäure oder deren Salzen, Oxybutynin, Bromelain und Aescin enthält.

5. Katheter (1) in Form eines Verweilkatheters zur medikamentösen und physikalischen Behandlung der Prostata (3) und gegebenenfalls der Blase (2), vorzugsweise in Verbindung mit oder nach einer transurethralen Resektion und/oder Vaporisation der Prostata (3), in Verbindung mit einer benignen Hyperplasie der Prostata (3) oder in Verbindung mit oder nach einer Bestrahlungstherapie eines Karzinoms der Prostata (3),
wobei der Katheter (1)
- einen Katheterschlauch (4),
- einen am Katheterschlauch (4) befindlichen Katheteranschluss (5') und
- einen befüllbaren und/oder expandierbaren Prostataballon (16),
aufweist,
wobei der Katheterschlauch (4) ein distales Ende (5) und ein proximales Ende (7) aufweist, wobei sich der Katheteranschluss (5') am distalen Ende (5) befindet,
wobei ein vom Katheteranschluss (5') bis zu wenigstens einer Ablauföffnung (10) im Bereich des proximalen Endes (7) des Katheterschlauchs (4) reichender erster Kanal (8) vorhanden ist,
wobei sich der Prostataballon (16) am proximalen Ende (7) des Katheterschlauchs (4) befindet,
wobei ein vom Katheteranschluss (5') bis zu dem Prostataballon (16) im Bereich des proximalen Endes (7) des Katheterschlauchs (4) reichender zweiter Kanal (15) vorhanden ist,
wobei der Prostataballon (16) eine mittels Poren (25) permeabel ausgebildete Wandung (17) aufweist, wobei der Durchmesser der Poren (25) eingestellt ist derart, dass im eingeführten Zustand des Katheters (1) eine gezielte Abgabe eines Medikaments (14) durch die Wandung (17) hindurch erfolgen kann, wobei die Wandung (17) des Prostataballons (16) eine Mehrzahl von Poren (25) mit einem Durchmesser im Bereich von 1 bis 100 µm im expandierten Zustand aufweist und wobei die Poren (25) durch Laserperforation erzeugt sind, und
wobei im Bereich des proximalen Endes (7) des Katheterschlauchs (4) Mittel (12) zur Positionierung des proximalen Endes (7) vorhanden sind, wobei als Mittel (12) zur Positionierung des proximalen Endes (7) ein expandierbarer und/oder befüllbarer Blasenballon (12) vorhanden ist.

6. Katheter nach Anspruch 5, **dadurch gekennzeichnet,**
**dass** die Wandung (17) des Prostataballons (16) 2 bis 1.000 Poren (25), insbesondere 2 bis 500 Poren (25), vorzugsweise 2 bis 100 Poren (25), bevorzugt 2 bis 50 Poren (25), besonders bevorzugt 2 bis 20 Poren (25), ganz besonders bevorzugt 3 bis 10 Poren (25), insbesondere bevorzugt 4 bis 6 Poren (25), aufweist und/oder
**dass** die Wandung (17) des Prostataballons (16) im expandierten Zustand Poren (25) mit einem Durchmesser im Bereich von 2 bis 80 µm, vorzugsweise 3 bis 60 µm, bevorzugt 4 bis 50 µm, besonders bevorzugt 5 bis 40 µm, ganz besonders bevorzugt 8 bis 30 µm, noch mehr bevorzugt 15 bis 25 µm, aufweist und/oder
**dass** die Poren (25) durch Laserperforation mittels eines Ultrakurzpulslasers erzeugt sind.

7. Katheter nach Anspruch 5 oder 6, **dadurch gekennzeichnet,**
**dass** der Prostataballon (16) mindestens ein vorzugsweise organisches Polymer aufweist und/oder hieraus besteht und/oder
**dass** der Prostataballon (16) (i) Kautschuk, insbesondere Naturkautschuk, Isoprenkautschuk, Polyurethankautschuk, Acrylnitril-Butadien-Kautschuk und/oder Styrol-Butadien-Kautschuk; (ii) Polyurethane, insbesondere thermoplastische Polyurethane; (iii) Polyvinylchlorid (PVC); (iv) Latex und/oder (v) Silikon; vorzugsweise Silikon, aufweist und/oder hieraus besteht und/oder
**dass** die Wandung (17) des Prostataballons (16) im nichtexpandierten Zustand eine Dicke im Bereich von 50 bis 500 µm, insbesondere 100 bis 450 µm, vorzugsweise 150 bis 400 µm, bevorzugt 200 bis 300 µm, aufweist und/oder dass die Wandung (17) des Prostataballons (16) im expandierten Zustand eine Dicke im Bereich von 10 bis 250 µm, insbesondere 20 bis 200 µm, vorzugsweise 30 bis 150 µm, bevorzugt 40 bis 100 µm, aufweist.

8. Katheter nach einem der Ansprüche 5 bis 7, **dadurch gekennzeichnet,**
**dass** der Prostataballon (16) nach Befüllung und/oder im expandierten Zustand zylinderförmig ausgebildet ist und/oder dass der Prostataballon (16) eine Länge im Bereich zwischen 2 cm und 6 cm aufweist und/oder
**dass** der Prostataballon (16) im expandierten Zustand ein Volumen im Bereich von 10 bis 150 ml, insbesondere 15 bis 120 ml, vorzugsweise 20 bis 100 ml, aufweist, insbesondere wobei der Prostataballon (16) im expandierten Zustand ein Volumen im Bereich von 10 bis 50 ml, insbesondere 15 bis 45 ml, vorzugsweise 20 bis 40 ml, aufweist oder insbesondere wobei der Prostataballon (16) im expandierten Zustand ein Volumen im Bereich von 40 bis 80 ml, insbesondere 45 bis 75 ml, vorzugsweise 40 bis 70 ml, aufweist oder insbesondere wobei der Prostataballon (16) im expandierten Zustand ein Volumen im Bereich von 70 bis 120 ml, insbesondere 75 bis 110 ml, vorzugsweise 80 bis 100 ml, aufweist.

9. Katheter nach einem der Ansprüche 5 bis 8, **dadurch gekennzeichnet,**
**dass** der Prostataballon (16) mit mindestens einem Medikament (14), insbesondere in flüssiger oder fließfähiger Form, vorzugsweise in Form einer mindestens einen pharmazeutischen Wirkstoff enthaltenden flüssigen Zusammensetzung, befüllbar ausgebildet ist und/oder befüllt ist;
insbesondere wobei das Medikament (14) in viskoser und/oder gelförmiger Form ausgebildet ist und/oder in Form eines Gels vorliegt und/oder
insbesondere wobei das Medikament (14) bei einer Temperatur von 25 °C eine Brookfield-Viskosität im Bereich von 500 bis 100.000 mPas, insbesondere 750 bis 10.000 mPas, bevorzugt 1.000 bis 7.500 mPas, besonders bevorzugt 1.250 bis 5.000 mPas, ganz besonders bevorzugt 1.300 bis 3.000 mPas, aufweist und/oder
insbesondere wobei das Medikament (14) bei einer Temperatur von 25 °C und bei Atmosphärendruck (1.013 hPa) eine Dichte im Bereich von 0,7 bis 1,5 g/cm³, insbesondere 0,8 bis 1,4 g/cm³, bevorzugt 0,85 bis 1,3 g/cm³, besonders bevorzugt 0,9 bis 1,2 g/cm³, ganz besonders bevorzugt 0,95 bis 1,1 g/cm³, aufweist; und/oder
**dass** der Prostataballon (16) zusätzlich beschichtet ist, insbesondere mit mindestens einem Medikament (14), und/oder dass auf den Prostataballon (16) zusätzlich mindestens ein Medikament (14) aufgebracht ist und/oder
**dass** mindestens ein Medikament (14) in die Wandung (17) des Prostataballons (16) eingelagert und/oder eingebracht ist und/oder dass mindestens ein Medikament (14) an die Wandung (17) des Prostataballons (16) angelagert und/oder angebunden und/oder hiermit verbunden ist.

10. Katheter nach einem der Ansprüche 5 bis 9, **dadurch gekennzeichnet,**
**dass** der Katheter (1), insbesondere der Katheterschlauch (4), einen äußeren Durchmesser im Bereich von 12 bis 30 Charr., insbesondere 14 bis 28 Charr., vorzugsweise 16 bis 26 Charr., bevorzugt 18 bis 24 Charr., aufweist und/oder
**dass** der Katheterschlauch (4) einen äußeren Mantel (6), insbesondere auf Basis von Silikon, aufweist und/oder
**dass** der Katheter (1) eine Gesamtlänge im Bereich von 10 bis 100 cm, insbesondere 15 bis 50 cm, vorzugsweise 20 bis 40 cm, aufweist und/oder
**dass** am proximalen Ende (7) des Katheterschlauchs (4) eine endseitig geschlossene Spitze (20) vorhanden ist, insbesondere wobei das Ende der Spitze (20) abgerundet ausgebildet ist und/oder insbesondere wobei die Spitze (20) gebogen ausgebildet ist und/oder insbesondere wobei die Spitze (20) die mindestens eine Ablauföffnung (10), insbesondere mindestens zwei Ablauföffnungen (10), insbesondere zum Ablassen von Urin (9) aus der Blase (2), aufweist, und/oder
**dass** die Ablauföffnung (10) kreis- oder schlitzförmig, insbesondere schlitzförmig, ausgebildet ist und/oder
**dass** eine Mehrzahl von Ablauföffnungen (10), insbesondere mindestens zwei Ablauföffnungen (10), vorhanden ist.

11. Katheter nach einem der Ansprüche 5 bis 10, **dadurch gekennzeichnet,**
**dass** der Blasenballon (12) im expandierten Zustand und/oder nach Befüllung kugelförmig ausgebildet ist und/oder
**dass** ein vom Katheteranschluss (5') bis zu dem befüllbaren und/oder expandierbaren Blasenballon (12), insbesondere in den Bereich des proximalen Endes (7) des Katheterschlauchs (4), reichender dritter Kanal (11) vorhanden ist und/oder
**dass** der lichte Abstand (a) des Prostataballons (16) zum Blasenballon (12) wenigstens 0,5 cm und vorzugsweise 1,0 cm bis 3,0 cm, insbesondere etwa 1,5 cm, beträgt und/oder
**dass** der Blasenballon (12) mindestens ein vorzugsweise organisches Polymer aufweist und/oder hieraus besteht und/oder
**dass** der Blasenballon (12) (i) Kautschuk, insbesondere Naturkautschuk, Isoprenkautschuk, Polyurethankautschuk, Acrylnitril-Butadien-Kautschuk und/oder Styrol-Butadien-Kautschuk; (ii) Polyurethane, insbesondere thermoplastische Polyurethane; (iii) Polyvinylchlorid (PVC); (iv) Latex und/oder (v) Silikon; vorzugsweise Silikon, aufweist und/oder hieraus besteht und/oder
**dass** der Blasenballon (12) im expandierten Zustand ein Volumen im Bereich von 10 bis 200 ml, insbesondere 15 bis 150 ml, vorzugsweise 20 bis 100 ml, vorzugsweise 25 bis 75 ml, aufweist und/oder
**dass** die Wandung (13) des Blasenballons (12) im nichtexpandierten Zustand eine Dicke im Bereich von 50 bis 500 µm, insbesondere 100 bis 450 µm, vorzugsweise 150 bis 400 µm, bevorzugt 200 bis 300 µm, aufweist und/oder dass die Wandung (13) des Blasenballons (12) im expandierten Zustand eine Dicke im Bereich von 10 bis 250 µm, insbesondere 20 bis 200 µm, vorzugsweise 30 bis 150 µm, bevorzugt 40 bis 100 µm, aufweist und/oder
**dass** der Blasenballon (12) eine Wandung (13) aufweist und/oder von einer Wandung (13) begrenzt wird, insbesondere wobei die Wandung (13) impermeabel, insbesondere medikamentenundurchlässig, ausgebildet ist oder insbesondere wobei die Wandung (13) permeabel, insbesondere medikamentendurchlässig, ausgebildet ist.

12. Katheter nach einem der Ansprüche 5 bis 11, **dadurch gekennzeichnet,**
**dass** der Blasenballon (12) eine permeabel, insbesondere mittels Poren (26) permeabel, vorzugsweise medikamentendurchlässig, ausgebildete Wandung (13) aufweist;
insbesondere wobei die Wandung (13) des Blasenballons (12) eine Mehrzahl von Poren (26) aufweist und/oder
insbesondere wobei die Wandung (13) des Blasenballons (12) 1 bis 1.000 Poren (26), insbesondere 1 bis 500 Poren (26), vorzugsweise 2 bis 100 Poren (26), bevorzugt 2 bis 50 Poren (26), besonders bevorzugt 2 bis 20 Poren (26), ganz besonders bevorzugt 3 bis 10 Poren (26), insbesondere bevorzugt 4 bis 6 Poren (26), aufweist und/oder
insbesondere wobei die Wandung (13) des Blasenballons (12) im expandierten Zustand Poren (26) mit einem Durchmesser im Bereich von 1 bis 100 µm, insbesondere 2 bis 80 µm, vorzugsweise 3 bis 60 µm, bevorzugt 4 bis 50 µm, besonders bevorzugt 5 bis 40 µm, ganz besonders bevorzugt 8 bis 30 µm, noch mehr bevorzugt 15 bis 25 µm, aufweist und/oder
insbesondere wobei die Poren (26) durch Laserperforation, insbesondere mittels eines Ultrakurzpulslasers, erzeugt sind.

13. Katheter nach einem der Ansprüche 5 bis 12, **dadurch gekennzeichnet, dass** der Blasenballon (12) mit mindestens einem Medikament (14), insbesondere in flüssiger oder fließfähiger Form, vorzugsweise in Form einer mindestens einen pharmazeutischen Wirkstoff enthaltenden flüssigen Zusammensetzung, befüllbar ausgebildet ist und/oder befüllt ist;
insbesondere wobei das Medikament (14) in viskoser Form ausgebildet ist und/oder in Form eines Gels vorliegt und/oder
insbesondere wobei das Medikament (14) bei einer Temperatur von 25 °C eine Brookfield-Viskosität im Bereich von 500 bis 100.000 mPas, insbesondere 750 bis 10.000 mPas, bevorzugt 1.000 bis 7.500 mPas, besonders bevorzugt 1.250 bis 5.000 mPas, ganz besonders bevorzugt 1.300 bis 3.000 mPas, aufweist und/oder
insbesondere wobei das Medikament (14) bei einer Temperatur von 25 °C und bei Atmosphärendruck (1.013 hPa) eine Dichte im Bereich von 0,7 bis 1,5 g/cm³, insbesondere 0,8 bis 1,4 g/cm³, bevorzugt 0,85 bis 1,3 g/cm³, besonders bevorzugt 0,9 bis 1,2 g/cm³, ganz besonders bevorzugt 0,95 bis 1,1 g/cm³, aufweist.

14. Katheter nach einem der Ansprüche 5 bis 13, **dadurch gekennzeichnet,**
**dass** der Katheteranschluss (5') mindestens zwei, vorzugsweise mindestens drei, insbesondere drei, Anschlussenden am distalen Ende (5) aufweist, insbesondere ein Anschlussende des ersten Kanals (8), ein Anschlussende des zweiten Kanals (15) und ein Anschlussende des dritten Kanals (11) und/oder
**dass** der erste, zweite und dritte Kanal (8, 15, 11) mit einem Verschlusselement (22, 24, 23), insbesondere in Form eines Stopfens, Stöpsels, Ventils oder dergleichen, jeweils verschließbar ausgebildet sind, insbesondere wobei der erste Kanal (8) mit einem Stöpsel (22), gegebenenfalls mit Ventilmechanismus, verschließbar ausgebildet ist und/oder der zweite und dritte Kanal (15, 11) jeweils mit einem Ventil (24, 23) verschließbar ausgebildet sind und/oder
**dass** der zweite Kanal (15) über eine Auslassöffnung (19) mit dem Prostataballon (16) verbunden ist oder hierin mündet und/oder dass der Prostataballon (16) über eine Auslassöffnung (19) im zweiten Kanal (15) expandierbar und/oder befüllbar ist und/oder dass die Wandung (17) des Prostataballons (16) an den äußeren Rändern mit dem Katheterschlauch (4), insbesondere mit dem Mantel (6), umlaufend verbunden ist und auf diese Weise einen Ringraum ausbildet, in welchen eine Auslassöffnung (19) des zweiten Kanals (15) mündet und/oder
**dass** der dritte Kanal (11) über eine Auslassöffnung (18) mit dem Blasenballon (12) verbunden ist oder hierin mündet und/oder dass der Blasenballon (12) über eine Auslassöffnung (18) im dritten Kanal (11) expandierbar und/oder befüllbar ist und/oder dass die Wandung (13) des Blasenballons (12) an den äußeren Rändern mit dem Katheterschlauch (4), insbesondere mit dem Mantel (6), umlaufend verbunden ist und auf diese Weise einen Ringraum ausbildet, in welchen eine Auslassöffnung (18) des dritten Kanals (11) mündet.

## Claims

1. A kit (kit-of-parts or set) (28) in the form of a catheter system for medicinally and physically treating the prostate (3) and optionally the bladder (2), preferentially in conjunction with or after a transurethral resection and/or vaporisation of the prostate (3), in conjunction with a benign hyperplasia of the prostate (3) or in conjunction with or after a radiation therapy of a carcinoma of the prostate (3),
wherein the kit (28) comprises:
• on the one hand a catheter (1) in the form of an indwelling catheter,
wherein the catheter (1) comprises
- a catheter tube (4),
- a catheter connector (5') disposed on the catheter tube (4) and
- a fillable and/or expandable prostate balloon (16),
wherein the catheter tube (4) has a distal end (5) and a proximal end (7), wherein the catheter connector (5') is disposed at the distal end (5), wherein a first duct (8) reaching from the catheter connector (5') to at least one discharge opening (10) is provided in the region of the proximal end (7) of the catheter tube (4), wherein the prostate balloon (16) is disposed at the proximal end (7) of the catheter tube (4), wherein a second duct (15) reaching from the catheter connector (5') to the prostate balloon (16) is provided in the region of the proximal end (7) of the catheter tube (4),
wherein the prostate balloon (16) has a wall (17) that is permeable by means of pores (25), wherein the diameter of the pores (25) is such that in the inserted state of the catheter (1) a medicament (14) can be selectively discharged through the wall (17), wherein the wall (17) of the prostate balloon (16) has a plurality of pores (25) with a diameter in the range of from 1 to 100 µm in the expanded state, and wherein the pores (25) are produced by laser perforation, and
wherein means (12) for positioning the proximal end (7) are provided in the region of the proximal end (7) of the catheter tube (4), wherein an expandable and/or fillable bladder balloon (12) is provided as means (12) for positioning the proximal end (7);
and
• on the other hand at least one application device (27) containing at least one medicament (14) for administering the medicament (14), in particular in the form of a preferentially sterile syringe, preferably a piston syringe, in particular for filling the catheter (1), in particular the prostate balloon (16), and as applicable the bladder balloon (12), with the medicament (14),
wherein the medicament (14) contains at least one pharmaceutical active substance, wherein the pharmaceutical active substance is selected from the group of antiphlogistics (anti inflammatories), anti-oedematous drugs and wound healing products and also mixtures and combinations thereof.

2. The kit according to claim 1, **characterised in that**
the pharmaceutical active substance is selected from the group of
(i) antiphlogistics, selected from serrapeptase, allantoin, coxibe, acetylsalicylic acid, ibuprofen, corticosteroids, in particular hydrocortisone, prednisolone, methylprednisolone, budesonide and/or dexamethasone, and diclofenac and also mixtures and combinations thereof, preferentially allantoin;
(ii) anti-oedematous drugs, selected from bromelain and aescin and also mixtures and combinations thereof;
(iii) spasmolytics, in particular selected from butylscopolamine (buscopan), hyoscyamine, scopolamine and ipratropium and also mixtures and combinations thereof;
(iv) anticholinergics, in particular selected from trospium chloride, tolterodine, darifenacin, solifenacin, oxybutynin and propiverine and also mixtures and combinations thereof, preferentially trospium chloride;
(v) wound healing products, selected from dexpanthenol and esters thereof, chondroitin and salts thereof, in particular chondroitin sulphate, onion extract, comfrey extract, hyaluronic acid and salts thereof, bisabolol, marigold extract, chamomile extract and witch hazel extract and also mixtures and combinations thereof, preferentially dexpanthenol, chondroitin and salts thereof and hyaluronic acid and salts thereof;
(vi) antiseptics, in particular selected from chlorohexidine, octenidine and polyhexanide and also mixtures and combinations thereof, preferentially polyhexanide;
(vii) local anaesthetics, in particular lidocaine;
and also mixtures and combinations thereof and/or
**in that** the medicament (14) has a content of active substance(s) in the range of from 0.00001 to 80 % by weight, in particular 0.0001 to 50 % by weight, preferentially 0.001 to 25 % by weight, preferably 0.005 to 20 % by weight, particularly preferably 0.01 to 10 % by weight, in relation to the medicament (14), and/or
**in that** the medicament (14), besides the active substance, also contains a pharmaceutically compatible excipient, in particular water or a preferentially isotonic aqueous table salt solution, in particular Ringer's solution, in particular in amounts in the range of from 20 to 99.99999 % by weight, in particular 50 to 99.9999 % by weight, preferentially 75 to 99.999 % by weight, preferably 80 to 99.995 % by weight, particularly preferably 90 to 99.99 % by weight, in relation to the medicament (14).

3. The kit according to claim 1 or 2, **characterised in that**
the medicament (14) contains a combination of at least two, preferentially at least three different pharmaceutical active substances, wherein the combination comprises at least one wound healing product together with at least one antiseptic and/or at least one antiphlogistic (anti-inflammatory), preferentially dexpanthenol together with polyhexanide and/or allantoin, particularly preferably dexpanthenol together with polyhexanide and optionally allantoin;
in particular wherein the medicament (14), in relation to the medicament (14), contains wound healing product, in particular dexpanthenol, in amounts of from 0.001 to 50 % by weight, in particular 0.01 to 30 % by weight, preferentially 0.1 to 20 % by weight, preferably 0.5 to 10 % by weight, particularly preferably 1 to 6 % by weight,
in particular wherein the medicament (14), in relation to the medicament (14), contains antiseptic, in particular polyhexanide, in amounts of from 0.0001 to 20 % by weight, in particular 0.001 to 10 % by weight, preferentially 0.01 to 5 % by weight, preferably 0.05 to 2 % by weight, particularly preferably 0.05 to 1 % by weight,
in particular wherein the medicament (14), in relation to the medicament (14), contains antiphlogistic (anti-inflammatory), in particular allantoin, in amounts of from 0.0001 to 20 % by weight, in particular 0.001 to 10 % by weight, preferentially 0.01 to 5 % by weight, preferably 0.05 to 2 % by weight, particularly preferably 0.05 to 1 % by weight.

4. The kit according to any one of the preceding claims, **characterised in that** the medicament (14) contains a combination of at least two, preferentially at least three, different pharmaceutical active substances, wherein the combination contains at least two active substances from the group of dexpanthenol, polyhexanide, octenidine, chlorohexidine, allantoin, chondroitin or chondroitin sulphate, hyaluronic acid or salts thereof, oxybutynin, bromelain and aescin, and/or wherein the combination contains at least one wound healing product, preferentially dexpanthenol, together with at least one further active substance from the group of polyhexanide, octenidine, chlorohexidine, allantoin, chondroitin or chondroitin sulphate, hyaluronic acid or salts thereof, oxybutynin, bromelain and aescin.

5. A catheter (1) in the form of an indwelling catheter for medicinally and physically treating the prostate (3) and optionally the bladder (2), preferentially in conjunction with or after a transurethral resection and/or vaporisation of the prostate (3), in conjunction with a benign hyperplasia of the prostate (3) or in conjunction with or after a radiation therapy of a carcinoma of the prostate (3),
wherein the catheter (1) comprises
- a catheter tube (4),
- a catheter connector (5') disposed on the catheter tube (4) and
- a fillable and/or expandable prostate balloon (16),
wherein the catheter tube (4) has a distal end (5) and a proximal end (7), wherein the catheter connector (5') is disposed at the distal end (5),
wherein a first duct (8) reaching from the catheter connector (5') to at least one discharge opening (10) is provided in the region of the proximal end (7) of the catheter tube (4),
wherein the prostate balloon (16) is disposed at the proximal end (7) of the catheter tube (4),
wherein a second duct (15) reaching from the catheter connector (5') to the prostate balloon (16) is provided in the region of the proximal end (7) of the catheter tube (4),
wherein the prostate balloon (16) has a wall (17) that is permeable by means of pores (25), wherein the diameter of the pores (25) is such that in the inserted state of the catheter (1) a medicament (14) can be selectively discharged through the wall (17), wherein the wall (17) of the prostate balloon (16) has a plurality of pores (25) with a diameter in the range of from 1 to 100 µm in the expanded state, and wherein the pores (25) are produced by laser perforation, and
wherein means (12) for positioning the proximal end (7) are provided in the region of the proximal end (7) of the catheter tube (4), wherein an expandable and/or fillable bladder balloon (12) is provided as means (12) for positioning the proximal end (7).

6. The catheter according to claim 5, **characterised in that**
the wall (17) of the prostate balloon (16) has 2 to 1,000 pores (25), in particular 2 to 500 pores (25), preferentially 2 to 100 pores (25), preferably 2 to 50 pores (25), particularly preferably 2 to 20 pores (25), very particularly preferably 3 to 10 pores (25), in particular preferably 4 to 6 pores (25), and/or
**in that** the wall (17) of the prostate balloon (16) in the expanded state has pores (25) with a diameter in the range of from 2 to 80 µm, preferentially 3 to 60 µm, preferably 4 to 50 µm, particularly preferably 5 to 40 µm, very particularly preferably 8 to 30 µm, even more preferably 15 to 25 µm, and/or
**in that** the pores (25) are generated by laser perforation by means of an ultra-short-pulse laser.

7. The catheter according to claim 5 or 6, **characterised in that**
the prostate balloon (16) comprises a preferentially organic polymer and/or consists hereof, and/or
**in that** the prostate balloon (16) comprises and/or consists of (i) rubber, in particular natural rubber, isoprene rubber, polyurethane rubber, acrylonitrile-butadiene rubber and/or styrene-butadiene rubber; (ii) polyurethanes, in particular thermoplastic polyurethanes; (iii) polyvinyl chloride (PVC); (iv) latex and/or (v) silicone; preferentially silicone, and/or
**in that** the wall (17) of the prostate balloon (16) in the non-expanded state has a thickness in the range of from 50 to 500 µm, in particular 100 to 450 µm, preferentially 150 to 400 µm, preferably 200 to 300 µm, and/or **in that** the wall (17) of the prostate balloon (16) in the expanded state has a thickness in the range of from 10 to 250 µm, in particular 20 to 200 µm, preferentially 30 to 150 µm, preferably 40 to 100 µm.

8. The catheter according to any one of claims 5 to 7, **characterised in that**
the prostate balloon (16) after filling and/or in the expanded state is cylindrical, and/or **in that** the prostate balloon (16) has a length in the range between 2 cm and 6 cm, and/or
**in that** the prostate balloon (16) in the expanded state has a volume in the range of from 10 to 150 ml, in particular 15 to 120 ml, preferentially 20 to 100 ml, in particular wherein the prostate balloon (16) in the expanded state has a volume in the range of from 10 to 50 ml, in particular 15 to 45 ml, preferentially 20 to 40 ml, or in particular wherein the prostate balloon (16) in the expanded state has a volume in the range of from 40 to 80 ml, in particular 45 to 75 ml, preferentially 40 to 70 ml, or in particular wherein the prostate balloon (16) in the expanded state has a volume in the range of from 70 to 120 ml, in particular 75 to 110 ml, preferentially 80 to 100 ml.

9. The catheter according to any one of claims 5 to 8, **characterised in that**
the prostate balloon (16) can be filled and/or is filled with at least one medicament (14), in particular in liquid or flowable form, preferentially in the form of a liquid composition containing at least one pharmaceutical active substance;
in particular wherein the medicament (14) is in viscous and/or gel-like form and/or is provided in the form of a gel, and/or
in particular wherein the medicament (14) at a temperature of 25°C has a Brookfield viscosity in the range of from 500 to 100,000 mPas, in particular 750 to 10,000 mPas, preferably 1,000 to 7,500 mPas, particularly preferably 1,250 to 5,000 mPas, very particularly preferably 1,300 to 3,000 mPas, and/or
in particular wherein the medicament (14) at a temperature of 25°C and at atmospheric pressure (1,013 hPa) has a density in the range of from 0.7 to 1.5 g/cm³, in particular 0.8 to 1.4 g/cm³, preferably 0.85 to 1.3 g/cm³, particularly preferably 0.9 to 1.2 g/cm³, very particularly preferably 0.95 to 1.1 g/cm³; and/or
**in that** the prostate balloon (16) is additionally coated, in particular with at least one medicament (14), and/or **in that** at least one medicament (14) is additionally applied to the prostate balloon (16), and/or
**in that** at least one medicament (14) is incorporated and/or introduced into the wall (17) of the prostate balloon (16), and/or **in that** at least one medicament (14) is taken up and/or adsorbed on and/or connected to the wall (17) of the prostate balloon (16).

10. The catheter according to any one of claims 5 to 9, **characterised in that**
the catheter (1), in particular the catheter tube (4), has an outer diameter in the range of from 12 to 30 Charr., in particular 14 to 28 Charr., preferentially 16 to 26 Charr., preferably 18 to 24 Charr., and/or
**in that** the catheter tube (4) has an outer jacket (6), in particular based on silicone, and/or
**in that** that catheter (1) has an overall length in the range of from 10 to 100 cm, in particular 15 to 50 cm, preferentially 20 to 40 cm, and/or
**in that** a tip (20) which is closed at the end is provided at the proximal end (7) of the catheter tube (4), in particular wherein the end of the tip (20) is rounded, and/or wherein in particular the tip (20) is curved, and/or in particular wherein the tip (20) has the at least one discharge opening (10), in particular at least two discharge openings (10), in particular for draining urine (9) from the bladder (2), and/or
**in that** the discharge opening (10) is circular or slot-shaped, in particular slot-shaped, and/or
**in that** a plurality of discharge openings (10), in particular at least two discharge openings (10), is provided.

11. The catheter according to any one of claims 5 to 10, **characterised in that**
the bladder balloon (12) in the expanded state and/or after filling is spherical, and/or
**in that** a third duct (11) reaching in particular from the catheter connector (5') to the fillable and/or expandable bladder balloon (12), in particular into the region of the proximal end (7) of the catheter tube (4), is provided, and/or
**in that** the clear distance (a) of the prostate balloon (16) from the bladder balloon (12) is at least 0.5 cm and preferentially 1.0 cm to 3.0 cm, in particular approximately 1.5 cm,
and/or
**in that** the bladder balloon (12) comprises and/or consists of at least one, preferentially organic polymer, and/or
**in that** the bladder balloon (12) comprises and/or consists of (i) rubber, in particular natural rubber, isoprene rubber, polyurethane rubber, acrylonitrile-butadiene rubber and/or styrene-butadiene rubber; (ii) polyurethanes, in particular thermoplastic polyurethanes; (iii) polyvinyl chloride (PVC); (iv) latex and/or (v) silicone; preferentially silicone, and/or
**in that** the bladder balloon (12) in the expanded state has a volume in the range of from 10 to 200 ml, in particular 15 to 150 ml, preferentially 20 to 100 ml, preferentially 25 to 75 ml, and/or
**in that** the wall (13) of the bladder balloon (12) in the non-expanded state has a thickness in the range of from 50 to 500 µm, in particular 100 to 450 µm, preferentially 150 to 400 µm, preferably 200 to 300 µm, and/or **in that** the wall (13) of the bladder balloon (12) in the expanded state has a thickness in the range of from 10 to 250 µm, in particular 20 to 200 µm, preferentially 30 to 150 µm, preferably 40 to 100 µm, and/or
**in that** the bladder balloon (12) has a wall (13) and/or is delimited by a wall (13), in particular wherein the wall (13) is impermeable, in particular impermeable to medicaments, or in particular wherein the wall (13) is permeable, in particular is permeable to medicaments.

12. The catheter according to any one of claims 5 to 11, **characterised in that**
the bladder balloon (12) has a wall (13) that is permeable, in particular permeable by means of pores (26), preferentially permeable to medicaments,
in particular wherein the wall (13) of the bladder balloon (12) has a plurality of pores (26), and/or
in particular wherein the wall (13) of the bladder balloon (12) has 1 to 1,000 pores (26), in particular 1 to 500 pores (26), preferentially 2 to 100 pores (26), preferably 2 to 50 pores (26), particularly preferably 2 to 20 pores (26), very particularly preferably 3 to 10 pores (26), in particular preferably 4 to 6 pores (26), and/or
in particular wherein the wall (13) of the bladder balloon (12) in the expanded state has pores (26) with a diameter in the range of from 1 to 100 µm, in particular 2 to 80 µm, preferentially 3 to 60 µm, preferably 4 to 50 µm, particularly preferably 5 to 40 µm, very particularly preferably 8 to 30 µm, even more preferably 15 to 25 µm, and/or
in particular wherein the pores (26) are produced by laser perforation, in particular by means of an ultra-short-pulse laser.

13. The catheter according to any one of claims 5 to 12, **characterised in that** the bladder balloon (12) can be filled and/or is filled with at least one medicament (14), in particular in liquid or flowable form, preferentially in the form of a liquid composition containing at least one pharmaceutical active substance;
in particular wherein the medicament (14) is in viscous form and/or is provided in the form of a gel, and/or
in particular wherein the medicament (14) at a temperature of 25°C has a Brookfield viscosity in the range of from 500 to 100,000 mPas, in particular 750 to 10,000 mPas, preferably 1,000 to 7,500 mPas, particularly preferably 1,250 to 5,000 mPas, very particularly preferably 1,300 to 3,000 mPas, and/or
in particular wherein the medicament (14) at a temperature of 25°C and at atmospheric pressure (1,013 hPa) has a density in the range of from 0.7 to 1.5 g/cm³, in particular 0.8 to 1.4 g/cm³, preferably 0.85 to 1.3 g/cm³, particularly preferably 0.9 to 1.2 g/cm³, very particularly preferably 0.95 to 1.1 g/cm³.

14. The catheter according to any one of claims 5 to 13, **characterised in that**
the catheter connector (5') has at least two, preferentially at least three, in particular three, connector ends at the distal end (5), in particular a connector end of the first duct (8), a connector end of the second duct (15), and a connector end of the third duct (11), and/or
**in that** the first, second, and third duct (8, 15, 11) are each formed such that they can be closed by a closure element (22, 24, 23), in particular in the form of a stopper, plug, valve or the like, in particular wherein the first duct (8) is formed such that it can be closed by a plug (22), optionally with a valve mechanism, and/or the second and third duct (15, 11) are each formed such that they can be closed by a valve (24, 23), and/or
**in that** the second duct (15) is connected via an outlet opening (19) to the prostate balloon (16) or opens thereinto, and/or **in that** the prostate balloon (16) can be expanded and/or filled via an outlet opening (19) in the second duct (15), and/or **in that** the wall (17) of the prostate balloon (16) is connected peripherally at the outer edges to the catheter tube (4), in particular to the jacket (6), and in this way forms an annular space into which an outlet opening (19) of the second duct (15) opens, and/or
**in that** the third duct (11) is connected via an outlet opening (18) to the bladder balloon (12) or opens thereinto, and/or **in that** the bladder balloon (12) can be expanded and/or filled via an outlet opening (18) in the third duct (11), and/or **in that** the wall (13) of the bladder balloon (12) is connected peripherally at the outer edges to the catheter tube (4), in particular to the jacket (6), and in this way forms an annular space into which an outlet opening (18) of the third duct (11) opens.

## Revendications

1. Kit (kit ou ensemble de pièces) (28) sous la forme d'un système de cathéter pour le traitement médicamenteux et physique de la prostate (3) et éventuellement de la vessie (2), de préférence en association avec ou après une résection transurétrale et/ou une vaporisation de la prostate (3), en association avec une hyperplasie bénigne de la prostate (3) ou en association avec ou après une radiothérapie d'un carcinome de la prostate (3),
le kit (28) comprenant :
• d'une part un cathéter (1) sous forme d'un cathéter à demeure,
le cathéter (1)
- présentant un tube de cathéter (4),
- un raccord de cathéter (5') se trouvant sur le tube de cathéter (4) et
- un ballon prostatique (16) dilatable et/ou pouvant être rempli,
le tube de cathéter (4) présentant une extrémité distale (5) et une extrémité proximale (7), le raccord de cathéter (5') se trouvant au niveau de l'extrémité distale (5), un premier canal (8) partant du raccord de cathéter (5') et s'étendant jusqu'à au moins une ouverture d'écoulement (10) dans la zone de l'extrémité proximale (7) du tube de cathéter (4), le ballon prostatique (16) se trouvant à l'extrémité proximale (7) du tube de cathéter (4), un deuxième canal (15) partant du raccord de cathéter (5') et s'étendant jusqu'au ballon prostatique (16) dans la zone de l'extrémité proximale (7) du tube de cathéter (4),
le ballon prostatique (16) présentant une paroi (17) perméable au moyen de pores (25), le diamètre des pores (25) étant ajusté de telle sorte que à l'état inséré du cathéter (1) une administration ciblée d'un médicament (14) peut être effectuée à travers la paroi (17), la paroi (17) du ballon prostatique (16) présentant une pluralité de pores (25) ayant un diamètre dans la plage comprise entre 1 et 100 µm à l'état dilaté et les pores (25) étant réalisés par perforation laser, et
des moyens (12) destinés à positionner l'extrémité proximale (7) se trouvant dans la zone de l'extrémité proximale (7) du tube de cathéter (4), un ballonnet vésical pouvant être rempli et/ou dilatable (12) étant présent en tant que moyen (12) destiné à positionner l'extrémité proximale (7) ;
et
• d'autre part au moins un dispositif d'application (27) contenant au moins un médicament (14) destiné à administrer le médicament (14), en particulier sous la forme d'une seringue de préférence stérile, de préférence une seringue à piston, en particulier destiné à remplir du médicament (14) le cathéter (1), en particulier du ballon prostatique (16) et éventuellement du ballonnet vésical (12),
le médicament (14) contenant au moins un principe actif pharmaceutique, le principe actif pharmaceutique étant sélectionné dans le groupe des antiphlogistiques (anti-inflammatoires), des anti-oedémateux et des préparations cicatrisante ainsi que leurs mélanges et combinaisons.

2. Kit selon la revendication 1, **caractérisé en ce que**
le principe actif pharmaceutique est sélectionné dans le groupe
(i) des antiphlogistiques, sélectionnés parmi la serrapeptase, l'allantoïne, le coxibe, l'acide acétylsalicylique, l'ibuprofène, les corticostéroïdes, en particulier l'hydrocortisone, la prednisolone, la méthylprednisolone, le budésonide et/ou la dexaméthasone, et le diclofénac et leurs mélanges et combinaisons, de préférence l'allantoïne ;
(ii) des anti-oedémateux, sélectionnés parmi le bromélaïne et l'aescine ainsi que leurs mélanges et combinaisons ;
(iii) des spasmolytiques, en particulier sélectionnés parmi la butylscopolamine (Buscopan), l'hyoscyamine, la scopolamine et l'ipratropium ainsi que leurs mélanges et combinaisons ;
(iv) des anticholinergiques, en particulier sélectionnés parmi le chlorure de trospium, la toltérodine, la darifénacine, la solifénacine, l'oxybutynine et la propivérine ainsi que leurs mélanges et combinaisons, de préférence le chlorure de trospium ;
(v) des préparations cicatrisantes, sélectionnées parmi le dexpanthénol et ses esters, la chondroïtine et ses sels, en particulier le sulfate de chondroïtine, l'extrait d'oignon, l'extrait de consoude, l'acide hyaluronique et ses sels, le bisabolol, l'extrait de calendula, l'extrait de camomille et l'extrait d'hamamélis ainsi que leurs mélanges et combinaisons, de préférence le dexpanthénol, la chondroïtine et ses sels et l'acide hyaluronique et ses sels ;
(vi) des antiseptiques, en particulier sélectionnés parmi la chlorhexidine, l'octénidine et le polyhexaméthylène biguanide ainsi que leurs mélanges et combinaisons, de préférence le polyhexaméthylène biguanide ;
(vii) des analgésiques locaux, en particulier la lidocaïne ;
ainsi que leurs mélanges et combinaisons et/ou
**en ce que** le médicament (14) présente une teneur en principe(s) actif(s) dans la plage comprise entre 0,00001 et 80 % en poids, en particulier entre 0,0001 et 50 % en poids, de préférence entre 0,001 et 25 % en poids, préférentiellement entre 0,005 et 20 % en poids, de préférence encore entre 0,01 et 10 % en poids, rapporté au médicament (14), et/ou
**en ce que** le médicament (14) contient, outre le principe actif, un excipient pharmaceutiquement compatible, en particulier de l'eau ou de préférence une solution saline aqueuse isotonique, en particulier le liquide de Ringer, en particulier dans des quantités dans la plage comprise entre 20 et 99,99999 % en poids, en particulier comprise entre 50 et 99,9999 % en poids, de préférence entre 75 et 99,999 % en poids, préférentiellement entre 80 et 99,995 % en poids, de préférence encore entre 90 et 99,99 % en poids, par rapport au médicament (14).

3. Kit selon la revendication 1 ou 2, **caractérisé en ce que**
le médicament (14) contient une combinaison d'au moins deux, de préférence d'au moins trois principes actifs pharmaceutiques différents les uns des autres, la combinaison comprenant au moins une préparation cicatrisante conjointement avec au moins un antiseptique et/ou au moins un antiphlogistique (anti-inflammatoire), de préférence le dexpanthénol conjointement avec le polyhexanide et/ou l'allantoïne, de préférence encore le dexpanthénol conjointement avec le polyhexaméthylène biguanide et éventuellement l'allantoïne ;
en particulier le médicament (14), contenant une préparation cicatrisante, en particulier du dexpanthénol, en des quantités comprises entre 0,001 et 50 % en poids, en particulier entre 0,01 et 30 % en poids, de préférence entre 0,1 et 20 % en poids, préférentiellement entre 0,5 et 10 % en poids, de préférence encore entre 1 et 6 % en poids, par rapport au médicament (14),
en particulier le médicament (14) contenant un antiseptique, en particulier du polyhexaméthylène biguanide, en des quantités comprises entre 0,0001 et 20 % en poids, en particulier entre 0,001 et 10 % en poids, de préférence entre 0,01 et 5 % en poids, de préférence entre 0,05 et 2 % en poids, de préférence encore entre 0,05 et 1 % en poids, par rapport au médicament (14),
en particulier le médicament (14) contenant un antiphlogistique (anti-inflammatoire), en particulier de l'allantoïne, en des quantités comprises entre 0,0001 et 20 % en poids, en particulier entre 0,001 et 10 % en poids, de préférence entre 0,01 et 5 % en poids, de préférence entre 0,05 et 2 % en poids, de préférence encore entre 0,05 et 1 % en poids, par rapport au médicament (14).

4. Kit selon l'une des revendications précédentes, **caractérisé en ce que** le médicament (14) contient une combinaison d'au moins deux, de préférence d'au moins trois principes actifs pharmaceutiques différents les uns des autres, la combinaison contenant au moins deux principes actifs du groupe consistant en le dexpanthénol, le polyhexaméthylène biguanide, l'octénidine, la chlorhexidine, l'allantoïne, la chondroïtine ou le sulfate de chondroïtine, l'acide hyaluronique ou ses sels, l'oxybutynine, la bromélaïne et l'aescine et/ou la combinaison contenant au moins une préparation cicatrisante, de préférence du dexpanthénol, conjointement avec au moins un autre principe actif du groupe consistant en le polyhexaméthylène biguanide, l'octénidine, la chlorhexidine, l'allantoïne, la chondroïtine ou le sulfate de chondroïtine, l'acide hyaluronique ou ses sels, l'oxybutynine, la bromélaïne et l'aescine.

5. Cathéter (1) sous forme d'un système de cathéter à demeure pour le traitement médicamenteux et physique de la prostate (3) et éventuellement de la vessie (2), de préférence en association avec ou après une résection transurétrale et/ou une vaporisation de la prostate (3), en association avec une hyperplasie bénigne de la prostate (3) ou en association avec ou après une radiothérapie d'un carcinome de la prostate (3),
le cathéter (1)
- présentant un tube de cathéter (4),
- un raccord de cathéter (5') se trouvant sur le tube de cathéter (4) et
- un ballon prostatique (16) dilatable et/ou pouvant être rempli,
le tube de cathéter (4) présentant une extrémité distale (5) et une extrémité proximale (7), le raccord de cathéter (5') se trouvant sur l'extrémité distale (5),
un premier canal (8) étant présent, partant du raccord de cathéter (5') et s'étendant jusqu'à au moins une ouverture d'écoulement (10) dans la zone de l'extrémité proximale (7) du tube de cathéter (4),
le ballon prostatique (16) se trouvant à l'extrémité proximale (7) du tube de cathéter (4),
un deuxième canal (15) étant présent, partant du raccord de cathéter (5') et s'étendant jusqu'au ballon prostatique (16) étant présent dans la zone de l'extrémité proximale (7) du tube de cathéter (4),
le ballon prostatique (16) présentant une paroi (17) perméable au moyen de pores (25), le diamètre des pores (25) étant ajusté de telle sorte que à l'état inséré du cathéter (1) une administration ciblée d'un médicament (14) peut être effectuée à travers la paroi (17), la paroi (17) du ballon prostatique (16) présentant une pluralité de pores (25) ayant un diamètre dans la plage comprise entre 1 et 100 µm à l'état dilaté et les pores (25) étant réalisés par perforation laser, et
des moyens (12) destinés à positionner l'extrémité proximale (7) se trouvant dans la zone de l'extrémité proximale (7) du tube de cathéter (4), un ballonnet vésical pouvant être rempli et/ou dilatable (12) étant présent en tant que moyen (12) destiné à positionner l'extrémité proximale (7) ;

6. Cathéter selon la revendication 5, **caractérisé**
**en ce que** la paroi (17) du ballon prostatique (16) présente de 2 à 1 000 pores (25), en particulier de 2 à 500 pores (25), de préférence de 2 à 100 pores (25), préférentiellement de 2 à 50 pores (25), de préférence encore de 2 à 20 pores (25), idéalement de 3 à 10 pores (25), d'une manière particulièrement préférée de 4 à 6 pores (25), et/ou
**en ce que** la paroi (17) du ballon prostatique (16) présente à l'état dilaté des pores (25) ayant un diamètre dans la plage comprise entre 2 et 80 µm, de préférence entre 3 et 60 µm, de préférence entre 4 et 50 µm, de préférence encore entre 5 et 40 µm, idéalement entre 8 et 30 µm, mieux encore entre 15 et 25 µm, et/ou
**en ce que** les pores (25) sont réalisés par perforation laser au moyen d'un laser à impulsions ultracourtes.

7. Cathéter selon la revendication 5 ou 6, **caractérisé en ce que**
**en ce que** le ballon prostatique (16) présente au moins un polymère de préférence organique et/ou en est constitué et/ou
**en ce que** le ballon prostatique (16) (i) présente un caoutchouc naturel, un caoutchouc isoprène, un caoutchouc polyuréthanne, un caoutchouc acrylonitrile-butadiène et/ou un caoutchouc styrène-butadiène ; (ii) des polyuréthannes, en particulier des polyuréthanes thermoplastiques ; (iii) du chlorure de polyvinyle (PVC) ; (iv) du latex et/ou (v) de la silicone ; de préférence de la silicone et/ou en est constitué et/ou
**en ce que** la paroi (17) du ballon prostatique (16) présente à l'état non dilaté une épaisseur dans la plage comprise entre 50 et 500 µm, en particulier entre 100 et 450 µm, de préférence entre 150 et 400 µm, préférentiellement entre 200 et 300 µm, et/ou **en ce que** la paroi (17) du ballon prostatique (16) à l'état dilaté présente une épaisseur dans la plage comprise entre 10 et 250 µm, en particulier entre 20 et 200 µm, de préférence entre 30 et 150 µm, préférentiellement entre 40 et 100 µm.

8. Cathéter selon l'une quelconque des revendications 5 à 7, **caractérisé**
**en ce que** le ballon prostatique (16) est cylindrique après le remplissage et/ou à l'état dilaté et/ou en ce que le ballon prostatique (16) présente une longueur dans la plage comprise entre 2 cm et 6 cm et/ou
**en ce que** le ballon prostatique (16) présente à l'état dilaté un volume dans la plage comprise entre 10 et 150 ml, en particulier entre 15 et 120 ml, de préférence entre 20 et 100 ml, en particulier le ballon prostatique (16) à l'état dilaté présentant un volume dans la plage comprise entre 10 et 50 ml, en particulier entre 15 et 45 ml, de préférence entre 20 et 40 ml, ou en particulier le ballon prostatique (16) présentant à l'état dilaté un volume compris dans la plage entre 40 et 80 ml, en particulier entre 45 et 75 ml, de préférence entre 40 et 70 ml, ou en particulier le ballon prostatique (16) à l'état dilaté présentant un volume dans la plage comprise entre 70 et 120 ml, en particulier entre 75 et 110 ml, de préférence entre 80 et 100 ml.

9. Cathéter selon l'une quelconque des revendications 5 à 8, caractérisé
le ballon prostatique (16) peut être rempli et/ou est rempli d'au moins un médicament (14), en particulier sous forme liquide ou fluide, de préférence sous forme d'une composition liquide contenant au moins un principe actif pharmaceutique ;
en particulier le médicament (14) étant visqueux et/ou gélifié et/ou se présentant sous forme de gel et/ou
en particulier, le médicament (14) présentant à une température de 25 °C une viscosité Brookfield dans la plage comprise entre 500 et 100 000 mPas, en particulier entre 750 et 10 000 mPas, de préférence entre 1 000 et 7 500 mPas, de préférence encore entre 1 250 et 5 000 mPas, idéalement entre 1 300 et 3 000 mPas, et/ou
en particulier le médicament (14) présentant à une température de 25 °C et sous la pression atmosphérique (1.013 hPa) une masse volumique dans la plage comprise entre 0,7 et 1,5 g/cm³, en particulier entre 0,8 et 1,4 g/cm³, de préférence entre 0,85 et 1,3 g/cm³, de préférence encore entre 0,9 et 1,2 g/cm³, idéalement entre 0,95 et 1,1 g/cm³, et/ou
en ce que le ballon prostatique (16) est en outre revêtu, en particulier par au moins un médicament (14), et/ou en ce que sur le ballon prostatique (16) est appliqué en outre au moins un médicament (14) et/ou
en ce qu'au moins un médicament (14) est incorporé et/ou inséré dans la paroi (17) du ballon prostatique (16) et/ou en ce qu'au moins un médicament (14) est fixé et/ou attaché à la paroi (17) du ballon prostatique (16) et/ou lui est relié.

10. Cathéter selon l'une quelconque des revendications 5 à 9, **caractérisé**
**en ce que** le cathéter (1), en particulier le tube de cathéter (4), présente un diamètre extérieur dans la plage comprise entre Ch. 12 et 30, en particulier entre Ch. 14 et 28, de préférence entre Ch. 16 et 26, de préférence entre Ch. 18 et 24, et/ou
**en ce que** le tube de cathéter (4) présente une enveloppe extérieure (6), en particulier à base de silicone et/ou
**en ce que** le cathéter (1) présente une longueur totale dans la plage comprise entre 10 et 100 cm, en particulier entre 15 et 50 cm, de préférence entre 20 et 40 cm, et/ou
**en ce que** sur l'extrémité proximale (7) du tube de cathéter (4) se trouve une seringue (20) fermée aux extrémités, de préférence l'extrémité de la seringue (20) étant arrondie et/ou en particulier la seringue (20) étant courbe et/ou en particulier la seringue (20) présentant l'au moins une ouverture d'écoulement (10), en particulier au moins deux ouvertures d'écoulement (10), en particulier pour évacuer l'urine (9) de la vessie (2), et/ou
**en ce que** l'ouverture d'écoulement (10) est circulaire ou en fente, en particulier en fente, et/ou
**en ce qu'**il existe une pluralité d'ouvertures d'écoulement (10), en particulier au moins deux ouvertures d'écoulement (10).

11. Cathéter selon l'une quelconque des revendications 5 à 10, **caractérisé**
**en ce que** le ballon prostatique (12) est sphérique à l'état dilaté et/ou après remplissage et/ou
**en ce qu'**un troisième canal (11) partant du raccord de cathéter (5') s'étend jusqu'au ballonnet vésical dilatable et/ou pouvant être rempli (12), en particulier dans la zone de l'extrémité proximale (7) du tube de cathéter (4), et/ou
**en ce que** l'écart libre (a) entre le ballon prostatique (16) et le ballonnet vésical (12) est d'au moins 0,5 cm et de préférence compris entre 1,0 cm et 3,0 cm, en particulier d'environ 1,5 cm, et/ou
**en ce que** le ballon prostatique (12) présente au moins un polymère de préférence organique et/ou en est constitué et/ou
**en ce que** le ballon prostatique (12) (i) présente un caoutchouc naturel, un caoutchouc isoprène, un caoutchouc polyuréthanne, un caoutchouc acrylonitrile-butadiène et/ou un caoutchouc styrène-butadiène ; (ii) des polyuréthannes, en particulier des polyuréthannes thermoplastiques ; (iii) du chlorure de polyvinyle (PVC) ; (iv) du latex et/ou (v) de la silicone ; de préférence de la silicone et/ou en est constitué et/ou
**en ce que** le ballonnet vésical (12) présente à l'état dilaté un volume dans la plage comprise entre 10 et 200 ml, en particulier entre 15 et 150 ml, de préférence entre 20 et 100 ml, préférentiellement entre 25 et 75 ml, et/ou
**en ce que** la paroi (13) du ballonnet vésical (12) présente à l'état non dilaté une épaisseur dans la plage comprise entre 50 et 500 µm, en particulier entre 100 et 450 µm, de préférence entre 150 et 400 µm, préférentiellement entre 200 et 300 µm, et/ou en ce que la paroi (13) du ballonnet vésical (12) présente à l'état dilaté une épaisseur dans la plage comprise entre 10 et 250 µm, en particulier entre 20 et 200 µm, de préférence entre 30 et 150 µm, de préférence entre 40 et 100 µm, et/ou
**en ce que** le ballonnet vésical (12) présente une paroi (13) et/ou est délimité par une paroi (13), en particulier la paroi (13) étant imperméable, en particulier étanche aux médicaments, ou en particulier la paroi (13) étant perméable, en particulier perméable aux médicaments.

12. Cathéter selon l'une quelconque des revendications 5 à 11, **caractérisé**
**en ce que** le ballonnet vésical (12) présente une paroi (13) perméable, en particulier perméable au moyen de pores (26) de préférence perméable aux médicaments ;
en particulier la paroi (13) du ballonnet vésical (12) présentant une pluralité de pores (26) et/ou
en particulier la paroi (13) du ballonnet vésical (12) présentant de 1 à 1 000 pores (26), en particulier de 1 à 500 pores (26), de préférence de 2 à 100 pores (26), préférentiellement de 2 à 50 pores (26), de préférence encore de 2 à 20 pores (26), idéalement de 3 à 10 pores (26), de préférence encore de 4 à 6 pores (26), et/ou
en particulier la paroi (13) du ballon de vessie (12) présentant à l'état dilaté des pores (26) ayant un diamètre dans la plage comprise entre 1 et 100 µm, en particulier entre 2 et 80 µm, de préférence entre 3 et 60 µm, préférentiellement entre 4 et 50 µm, de préférence encore entre 5 et 40 µm, mieux encore entre 8 et 30 µm, idéalement entre 15 et 25 µm et/ou
en particulier les pores (26) étant réalisés par perforation laser, en particulier au moyen d'un laser à impulsions ultracourtes.

13. Cathéter selon l'une quelconque des revendications 5 à 12, **caractérisé en ce que** le ballon prostatique (12) peut être rempli et/ou est rempli d'au moins un médicament (14), en particulier sous forme liquide ou fluide, de préférence sous forme d'une composition liquide contenant un principe actif pharmaceutique ;
en particulier le médicament (14) étant visqueux et/ou se présentant sous forme d'un gel et/ou
en particulier, le médicament (14) présentant à une température de 25 °C une viscosité Brookfield dans la plage comprise entre 500 et 100 000 mPas, en particulier entre 750 et 10 000 mPas, de préférence entre 1 000 et 7 500 mPas, de préférence encore entre 1 250 et 5 000 mPas, idéalement entre 1 300 et 3 000 mPas, et/ou
en particulier le médicament (14) présentant à une température de 25 °C et sous la pression atmosphérique (1013 hPa) une masse volumique dans la plage comprise entre 0,7 et 1,5 g/cm³, en particulier entre 0,8 et 1,4 g/cm³, de préférence entre 0,85 et 1,3 g/cm³, de préférence encore entre 0,9 et 1,2 g/cm³, idéalement entre 0,95 et 1,1 g/cm³.

14. Cathéter selon l'une quelconque des revendications 5 à 13, **caractérisé**
**en ce que** le raccord de cathéter (5') présente au moins deux, de préférence trois, en particulier trois extrémités de raccord à l'extrémité distale (5), en particulier une extrémité de raccord du premier canal (8), une extrémité de raccord du deuxième canal (15) et une extrémité de raccord du troisième canal (11) et/ou
**en ce que** le premier, le deuxième et le troisième canal (8, 15, 11) sont dotés d'un élément de fermeture (22, 24, 23), en particulier sous forme d'un obturateur, d'un bouchon, d'une soupape ou similaire, chacun étant refermable, en particulier le premier canal (8) étant refermable par un bouchon (22), éventuellement par un mécanisme de soupape et/ou le deuxième et le troisième canal (15, 11) étant chacun refermable par une soupape (24, 23) et/ou
**en ce que** le deuxième canal (15) est relié par une ouverture d'évacuation (19) au ballon prostatique (16) ou y débouchant et/ou en ce que le ballon prostatique (16) peut être dilaté et/ou rempli par une ouverture d'écoulement (19) aménagée dans le deuxième canal (15) et/ou en ce que la paroi (17) du ballon prostatique (16) est reliée sur toute la périphérie aux bords extérieurs au tube de cathéter (4), en particulier à l'enveloppe (6), et de cette manière forme une chambre annulaire, dans laquelle débouche une ouverture d'évacuation (19) du deuxième canal (15) et/ou
**en ce que** le troisième canal (11) est relié par une ouverture d'évacuation (18) au ballonnet vésical (12) ou y débouche et/ou en ce que le ballonnet vésical (12) peut être dilaté et/ou rempli par une ouverture d'évacuation (18) aménagée dans le troisième canal (11) et/ou en ce que la paroi (13) du ballonnet vésical (12) est reliée sur toute la périphérie sur les bords extérieurs au tube de cathéter (4), en particulier à l'enveloppe (6), et de cette manière forme une chambre annulaire dans laquelle débouche une ouverture d'évacuation (18) du troisième canal (11).
